# EUROPEAN PATENT SPECIFICATION

(11) **EP 3 840 701 B1**
(45) Date of publication and mention of the grant of the patent: **22.12.2021**
(21) Application number: 19763125.2
(22) Date of filing: 12.08.2019
(51) Int. Cl.: A61F 7/02, A61F 9/04

(54) **THERAPEUTIC EYE COMPRESS SYSTEM**
THERAPEUTISCHES AUGENKOMPRESSENSYSTEM
SYSTÈME DE COMPRESSE OCULAIRE THÉRAPEUTIQUE

(30) Priority: 21.08.2018 US 201816107200; 28.11.2018 US 201816202879; 27.03.2019 US 201916366310
(43) Date of publication of application: 30.06.2021
(60) Divisional application: 21205386.2
(73) Proprietor: Bruder Healthcare Company, LLC, Alpharetta, GA 30004 (US)
(72) Inventor: BRUDER, Mark H., Alpharetta, GA 30004 (US)
(74) Representative: Haseltine Lake Kempner LLP
(86) International application number: PCT/US2019/046128
(87) International publication number: WO 2020/041022

(56) References cited:
- WO-A1-2017/197496
- US-A1- 2014 330 222
- US-A1- 2017 252 210

## Description

### Technical Field

The present invention relates generally to the field of ophthalmic medical therapy or palliative care of the eye. More particularly, the present invention relates to a mask or compress for thermal treatment and/or delivery of medication to the eyes, and surrounding sinus and facial areas of a subject or patient. In particular applications, embodiments of the invention further relate to a moist heat delivery device with heat-loss preventive structures, thermal protective measures, and/or antimicrobial properties.

### Background

Various conditions of the eye may require medical or palliative care. For example, blepharitis is a common and ongoing condition where the eyelids become inflamed (swollen), with oily particles and bacteria coating the eyelid margin near the base of the eyelashes. This condition causes irritation, itchiness, redness, dry eye and stinging or burning of the eyes. While the underlying causes of blepharitis are not completely understood, it can be associated with a bacterial eye infection, symptoms of dry eyes or certain types of skin conditions such as acne rosacea. Anterior blepharitis affects the outside of the eyelid where the eyelashes are attached. This can be caused by bacterial (or sometimes viral) infection. If left untreated, anterior blepharitis can lead to thickened and inward-turned or outward-turned eyelids and even vision problems from in-turned eyelashes damaging the cornea. Posterior blepharitis is a condition that results from a dysfunction of the eye's tiny oil glands (meibomian glands) in the eyelids at the base of the eyelashes. When meibomian glands become clogged from posterior blepharitis, it can cause a stye or chalazion to form. Posterior blepharitis can also lead to thickened eyelid margins and crusty eyelids.

An estimated 40.9 million people in the United States aged 18 or older wear contact lenses. The International Workshop on Contact Lens Discomfort, published in 2013, put forth dryness of the eyes as a primary reason for contact lens intolerance. When a contact lens is placed on the eye, the tear film structure becomes altered resulting in a pre-lens thinned lipid layer and a post-lens thinned aqueous layer. As a result of this disruption from the contact lens, the tear film tends to have an increased rate of evaporation leading to poor wetting on the surface of the contact lens and inadequate lubrication on the surface of the eye. This is further exacerbated if the patient has an already unstable lipid layer due to the presence of meibomian gland dysfunction (MGD). MGD is considered by many to be the leading cause of dry eye disease throughout the world and is a chronic and progressive condition that can contribute to a poor quality lipid layer and lead to contact lens discomfort. Contact lens wearers often report dry eye symptoms and show signs of MGD including gland atrophy, thinned lipid layer, and increased tear film instability. It has been shown that in many patients with intolerance to contact lenses, MGD has been observed. Therefore, treatment of MGD may support the functioning of the meibomian glands and lead to improvement in patient contact lens comfort.

Hygienic home treatment of such ocular disorders can be a two-step process. First, the patient softens the debris and scurf that accumulates around the eye. The debris can be softened by applying a warm compress, diluted baby shampoo, or a specialized liquid solution to the eyelid margin. This first step is intended to prepare the debris for removal while preventing further irritation to the eye. Second, the patient can attempt to remove the debris by physically scrubbing the eyelid margin, the base of the eyelashes, and the pores of the meibomian glands. This scrubbing is routinely attempted with either a generic cotton swab, a fingertip, or a scrub pad placed over the fingertip and applied against the eye. By cleaning debris and scurf free from the base of the eyelashes and unclogging the pores of the meibomian glands, the patient may improve the overall health of the eyelid margin; thereby reducing irritation, burning, and other symptoms related to the disorder.

Thermal therapy can also be used for medical or palliative care of a human or animal subject or patient, for example by delivering moist heat or cold to the eye region. In example applications, thermal therapy can be used to unblock glands in the eye to help treat dry eye. Moist heat may also be used to help reduce elevated intraocular pressure to either treat or help prevent open-angle glaucoma. Delivery of medications to the eyes, such as for treatment of blepharitis may be enhanced by application of thermal therapy in combination with the medication. Applying heat to the inner eyelid may also help safely remove gland obstructions and stagnant gland content. Moist heat thermal therapy of the eyes may also be beneficial to extend comfortable wearing time of contact lenses.

Many currently known eye treatment masks are not designed to securely fit the eye, causing issues in some forms of therapeutic treatment. For example, when a patient uses a continuous positive airway pressure (CPAP) machine for treatment of sleep apnea, air can sometimes blowback from the mask of the CPAP machine into the user's eyes, causing dryness of the eyes. Known eye masks and eye compresses may not fit securely to the eye and have not been found entirely successful in protecting the eyes from this blow-back dryness.

Needs exist for improvements to ophthalmic medical therapy or palliative care of the eye. It is to the provision improved therapeutic eye mask system and treatment methods meeting these and other needs that the present invention is primarily directed.

### Summary

The invention is defined in the attached independent claim to which reference should now be made. Further optional features are defined in the sub-claims appended thereto. The invention as claimed relates to an eye compress configured to deliver moist heat therapy to at least one eye of a patient, including the further features as recited in the attached independent claim. The disclosure also extends to therapeutic mask systems, ophthalmic therapy devices, eye masks, and a method of providing moist heat therapy. Statements within this summary relate to features of such articles according to the disclosure, and should not be considered as mutually exclusive with features of the invention merely because all the features of the attached independent claim are not recited in those statements. The statements are useful for understanding the various constructions and arrangements that are envisaged by the disclosure, including an eye compress as defined in the attached independent claims.

The disclosure provides a therapeutic mask system for treatment of the eyes, including a mask portion for attachment to the head of a patient and an eye coverage pod that is detachably coupled to the mask portion. The mask portion can include an eye coverage portion comprising a receiver in which the pod is releasably secured. The pods can also be directly secured to a mask strap. The pod includes material for delivering thermal, moisture and/or medication therapy and treatment to the eye. The system can also include a heat-transmissive treatment pad positioned between the pod and the eye of the user.

The therapeutic device may include pods having a granular fill material such as a hydrophilic zeolite or molecular sieve material, optionally loaded with at least one metallic or other antimicrobial agent, such as for example a silver, copper, copper oxide, gold, magnesium oxide, aluminum oxide, titanium dioxide, zinc oxide, cobalt, nickel, zirconium, molybdenum, tin, lead and/or other metals, metal oxides, metal ions, metal particles or nanoparticles, and alloys, mixtures or combinations thereof. It may be that the antimicrobial agent is retained in the therapeutic device over multiple uses.

There is also disclosed an eye mask article or compress system comprising at least one eye coverage portion for application to at least one eye. The article includes a mask with at least one eye coverage portion for application to the at least one eye. The mask includes at least one indented pocket positioned to align with the at least one eye. The mask also includes at least one thermal treatment delivery pod detachably secured within the at least one indented pocket. The at least one pod includes material for delivering moist heat therapy and treatment to the at least one eye. The mask optionally further comprises medicament dispensed toward the at least one eye by the at least one pod.

It may be that the therapeutic device includes a mask body configured for attachment to a patient and at least one treatment pod comprising a loose, granular fill material contained within an outer shell. The at least one treatment pod is releasably coupled to the mask body.

According to the disclosure the ophthalmic therapy device comprises at least one therapy pod configured to cover the eye of the user and a sheet shaped to conform to the eye area of a patient. The sheet is impregnated with medication. The sheet is held between the at least one therapy pod and the eye. The therapy pod releases heat toward the eye and the medication is expelled from the sheet towards the eye.

The disclosure extends to a method which is outside of the scope of the attached claims which is useful for understanding applications of the articles disclosed herein, in particular a method of treating patient's eyes comprising removably attaching two pods to a therapeutic mask and applying the mask to an eye area of the patient. The pods comprise a heat delivery material.

The present disclosure provides a therapeutic eye mask or compress generally including at least one, and in particular embodiments two separate eye coverage portions - each configured to deliver moist heat therapy. The mask is configured to be worn on the head of a human or animal patient with one of the eye coverage portions positioned over at least one eye, and in particular embodiments over both eyes of the patient. Each eye coverage portion has a front side, configured to rest against the face of the patient, and a back side opposite the front side. In example embodiments, each eye coverage portion includes a moisture barrier material - positioned towards the back side of the eye coverage portion - configured to prevent moisture from escaping from the back side of the eye coverage portion, thereby directing delivery of moist heat therapy toward the patient.

The present disclosure provides an eye mask configured to deliver moist heat therapy to at least one eye of a patient - the eye mask comprising at least one eye coverage portion and at least one securing strap. Further the at least one eye coverage portion comprises a fill material configured to absorb and release moisture, such as water; a moisture-permeable material surrounding and containing the fill material; and a moisture-impermeable material attached to a surface of the moisture permeable material. The moisture-impermeable material is configured to at least partially contain and/or direct moisture released from the fill material.

The present disclosure provides an eye compress for delivery of moist heat to a patient. In example forms, the eye compress includes at least one enclosure containing a fill material capable of absorbing and releasing moist heat for therapeutic delivery to the patient, the enclosure having a first side and an opposite second side, with the fill material disposed between the first side and the second side, and wherein the first side is configured for application to a body portion of the patient. In example embodiments, the eye compress also includes a moisture barrier positioned along the second side, whereby moist heat is delivered from the fill material in a first direction toward and through the first side, and whereby moist heat is substantially prevented from transmission from the fill material in a second direction toward the second side.

The present disclosure provides a method that falls outside of the scope of the attached claims which is useful for understanding applications of the articles disclosed herein, in particular a method of providing moist heat therapy to a body part of a human or animal subject. In example forms, the method includes providing a therapeutic device comprising a first side, a second side, and a hydrophilic fill material contained between the first and second sides; wherein the first side at least partially comprises a moisture-transmissive material allowing passage of moisture therethrough for absorption and release into and from the fill material, and the second side at least partially comprises a moisture-impermeable material substantially preventing passage of moisture therethrough.

The present disclosure provides a therapeutic mask or compress system for treatment of the eyes, generally including at least one, and in particular embodiments two separate eye coverage portions, wherein each of the eye coverage portions is configured to deliver moist heat therapy to the eye area of a human or animal patient. The mask is configured to be worn on the head of the patient with one of the eye coverage portions positioned over at least one eye, and in particular optionally with eye coverage portions positioned over both eyes of the patient.

The disclosure provides an eye mask or compress according to the attached independent claim which comprises at least one eye coverage portion, at least one securing strap attached to the eye coverage portion(s) for securing the eye coverage portion(s) over the eye of the patient, and a fill material enclosed within each eye coverage portion. The eye coverage portions are formed from a moisture-permeable material comprising antimicrobial properties wherein the fill material is configured to absorb and release moisture through the moisture-permeable material. Each eye coverage portion has a front side, configured to rest against the face of the patient in contact with the area around one or both of the user's eyes, and a back side opposite the front side directed away from the user. According to the invention as claimed, the eye compress comprises a recessed area on at least the front side of the at least one eye coverage portion. It may be that each eye coverage portion also includes a generally centrally located dimple or recess on the front side or both sides, forming a retracted non-contact area configured allow space between the compress and the eye area of the subject, to prevent the application of excessive heat on and around the cornea, preventing conditions such as corneal warping, while allowing moist heat therapy to reach the eyelid and surrounding eye area.

The disclosure provides an eye compress for delivery of moist heat to a patient. In example forms, the eye compress includes at least one enclosure containing a fill material capable of absorbing and releasing moist heat for therapeutic delivery to the patient, the enclosure having a first side and an opposite second side, with the fill material disposed between the first and second sides, and wherein the first side is configured for application to an eye or other body portion of the patient. In example embodiments, the eye compress also includes a stitched or sutured segment joining the first side and the second side, wherein the stitched segment is configured to be positioned over a cornea of the eye of the patient, to prevent application of excess heat transmission from the fill material to the cornea of the patient or user.

The disclosure provides a method falling outside of the scope of the attached claims which is useful for understanding applications of the articles disclosed herein, in particular a method of providing moist heat therapy to an eye area of a human or animal subject. In example forms, the method includes providing a therapeutic device comprising at least one eye cover enclosure with a first side and a second side, a seam or stitched segment joining together the first and second sides to form a recessed non-contact area, and a hydrophilic fill material contained within the first and second sides of the eye cover enclosure. The first side of the eye cover enclosure at least partially comprises a moisture-transmissive material allowing passage of moisture therethrough for absorption into and release from the fill material. The stitched segment is configured to be positioned over the cornea of the eye of the human or animal subject to prevent excessive heat exposure to the cornea. Additionally, it may be that the eye cover enclosure at least partially comprises antimicrobial material. Document USA-2017/252210 discloses the most relevant prior art.

These and other aspects, features and advantages of the invention will be understood with reference to the drawing figures and detailed description herein, and will be realized by means of the various elements and combinations particularly pointed out in the appended claims. It is to be understood that both the foregoing general description and the following brief description of the drawings and detailed description are exemplary and explanatory of preferred embodiments of the invention, and are not restrictive of the invention, as claimed. The invention is defined in claim 1. Any embodiment which is in contradiction to the subject-matter of claim 1 is not part of the invention.

### Brief Description of the Drawings

In the following description, examples for which the associated description does not explicitly recite all features of the attached independent claim are indicated as comparative examples. Implementations of the invention as defined in the independent claim may nevertheless include features described with reference to the comparative examples. As various features applicable to implementations of the invention may be described herein with reference to the comparative examples, the description of the comparative examples is retained to aid the understanding of the invention.
FIGURE 1 is a perspective view of a therapeutic eye mask system according to a comparative example.
FIGURE 2 shows a back view of the therapeutic eye mask system depicted in Figure 1.
FIGURE 3 shows the therapeutic eye mask system depicted in Figure 2 including detachable pods.
FIGURES 4A-C show a front, back and side view of the therapeutic eye mask system of Figure 1.
FIGURE 5 is a perspective view of a therapeutic eye mask system according to another comparative example.
FIGURE 6 shows a back view of the therapeutic eye mask system of Figure 5.
FIGURES 7A-C show a front, back and side view of an eye mask portion for use in a therapeutic eye mask system according to another comparative example.
FIGURES 8A-C show a front, top and side views of a therapeutic eye mask system with the eye mask portion of Figures 7A-C.
FIGURE 9 shows the therapeutic eye mask of Figure 1 worn by a human patient.
FIGURE 10 shows the therapeutic eye mask of Figures 8A-C worn by a human patient.
FIGURES 11A-B show the therapeutic eye mask of Figures 8A-C worn by a human patient.
FIGURE 12 shows a therapeutic eye mask system according to another comparative example.
FIGURE 13 is an exploded view of a therapeutic eye mask system according to another comparative example, having interchangeable pods.
FIGURE 14 shows a detailed exploded view of the therapeutic eye mask system of Figure 13.
FIGURE 15 shows a detachable pod for use in a therapeutic eye mask according to a comparative example.
FIGURE 16 shows a detachable pod according to another comparative example.
FIGURE 17 shows detachable pods of varying shapes according to further comparative examples.
FIGURE 18 shows detachable pods of varying sizes according to various comparative examples.
FIGURE 19 shows a therapeutic eye mask system according to a comparative example.
FIGURE 20 shows a therapeutic eye mask system according to a comparative example.
FIGURE 21 is a perspective view of a therapeutic eye mask system according to an example embodiment of the invention.
FIGURE 22 shows a side view of the therapeutic eye mask system of Figure 21.
FIGURE 23 shows a back view of the therapeutic eye mask system of Figure 21.
FIGURE 24 is a cut-away view of a pod of the therapeutic eye mask system of Figure 21.
FIGURE 25 shows heat-transmissive pads for use with a therapeutic eye mask.
FIGURE 26 shows a side exploded view of a therapeutic eye mask system with heat-transmissive pads.
FIGURE 27 shows a therapeutic eye mask system according to another comparative example worn by a human patient.
FIGURE 28 shows a therapeutic eye mask system according to yet another example embodiment of the present invention.
FIGURE 29 is a side view of the therapeutic eye mask system of Figure 28.
FIGURE 30 shows a front view of the therapeutic eye mask system of Figure 28.
FIGURE 31 shows an alternative configuration of the therapeutic eye mask system of Figure 30.
FIGURE 32 shows a therapeutic eye mask system according to another example embodiment of the present invention.
FIGURE 33 is a perspective view of the therapeutic eye mask system of Figure 32.
FIGURE 34 is a cross-sectional view of the therapeutic eye mask system of Figure 33 at section line 34-34.
FIGURE 35 is a perspective view of a therapeutic eye mask according to another comparative example.
FIGURE 36 shows the therapeutic eye mask of Figure 35 worn by a human patient.
FIGURE 37 is a detailed view of the back side of an eye coverage portion of the therapeutic eye mask of Figure 35.
FIGURE 38 is a cutaway view of the eye coverage portion of Figure 37, wherein the outer cover layer is retracted.
FIGURE 39 is a cutaway view of the eye coverage portion of Figure 37, wherein the outer cover layer and intermediate layers are retracted.
FIGURE 40 is a cutaway view of the eye coverage portion of Figure 37, wherein the outer cover layer, intermediate layer, the moisture impermeable panel, and the inner pouch are retracted.
FIGURE 41 is a partial cross-section of an eye coverage portion of the therapeutic eye mask of Figure 35.
FIGURE 42 shows a detailed cross-section of the eye coverage portion of the therapeutic eye mask of Figure 35.

### Detailed Description

The present invention may be understood more readily by reference to the following detailed description taken in connection with the accompanying drawing figures, which form a part of this disclosure. It is to be understood that this invention is defined in the attached independent claim and is not limited to the specific devices, methods, conditions or parameters described and/or shown herein, and that the terminology used herein is for the purpose of describing particular embodiments by way of example only and is not intended to be limiting of the claimed invention.

Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

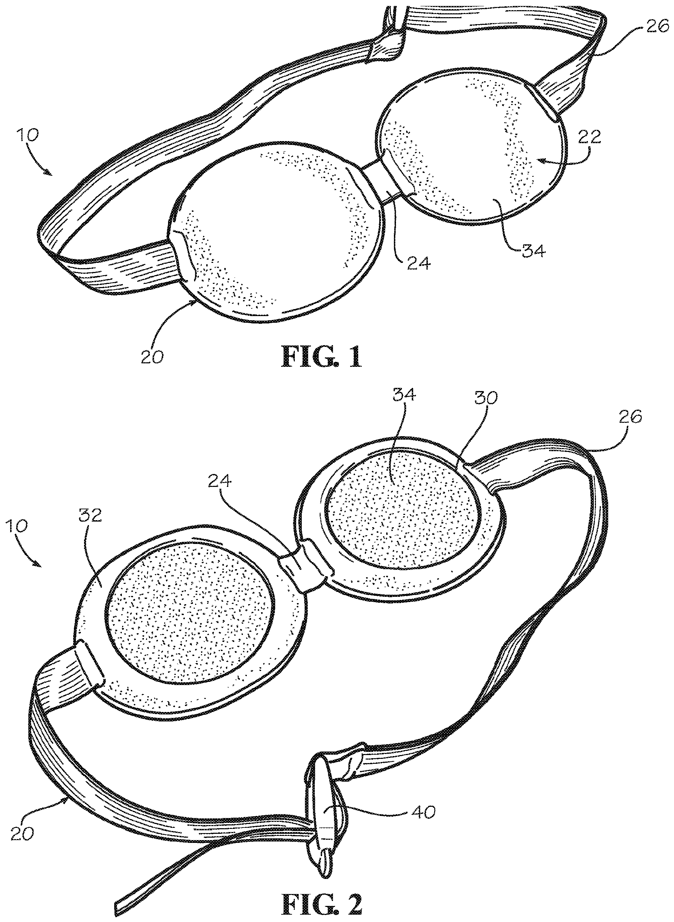

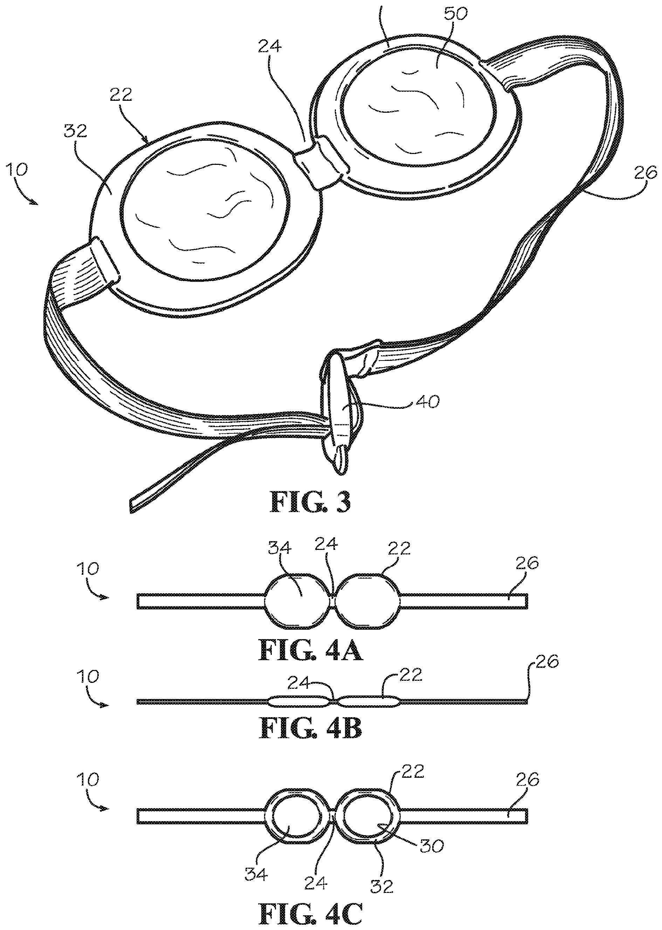

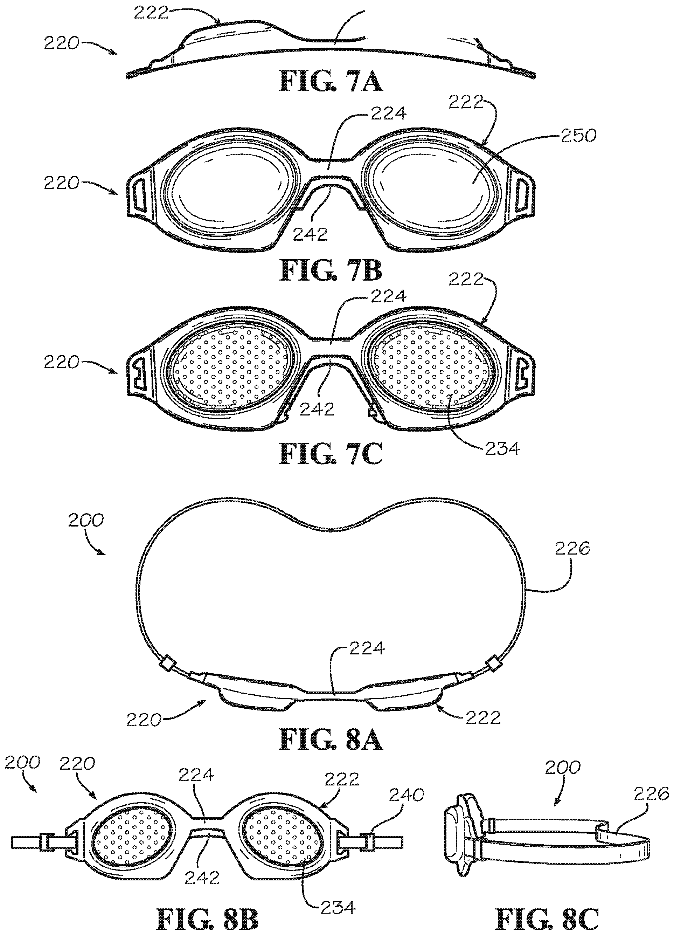

With reference now to the drawing figures, wherein like reference numbers represent corresponding parts throughout the several views, Figures 1-20 show a therapeutic eye mask system according to comparative examples described herein. The therapeutic eye mask system 10, shown in Figures 1-4, generally includes an eye mask 20 and at least one detachable therapy pod 50. The eye mask 20 can include two separate eye coverage portions 22 connected by a nose bridge 24 and a securing strap 26. The mask 20 is configured to be worn on the head of a human patient with one of the eye coverage portions 22 positioned over each eye of the patient. The securing strap 26 may be positioned around the user's head to hold the eye coverage portions 20 against the eyes of the user.

The mask 20 can have a monolithic uniform construction or may be separate pieces fastened together, for example by stitching, adhesive, fasteners or other attachment means. It may be that the mask 20 is constructed of a lightweight and durable material. The mask 20 can be made of a soft and flexible material, for example, foam or polyester. It can be constructed of perforated thermoformed foam to improve breathability. The mask 20 can be constructed from laminated foam or soft-flexible, open-cell foam with polyester fabric. In particular examples, the mask 20 is constructed from a 2 Ib./ft³, 3/8" thick polyether polyurethane foam, flame laminated to matte black polyester fiber interlock fabric on both sides. Alternatively, the mask 20 is constructed from polyester, rayon, spandex, silk or other natural and/or synthetic fabrics or materials. The mask material can optionally be selected to have insulative or heat-transmissive properties to affect the temperature transferred from the mask to the patient's eyes, ensuring safety. The material may optionally be washable for reuse, or alternatively can be a single-use disposable product. The mask 20 may be constructed of a material containing nanobeads comprising an antimicrobial metal.

The mask 20 depicted in Figures 1-4 includes two eye coverage portions 22. The eye coverage portions 22 are designed to be independent structures such that each can independently conform to the respective eye region of the patient. The eye coverage portions 22 can each include a receiver 30 for holding a detachable therapeutic pod 50. The receivers 30 are configured to hold the pods 50 in position over the patient's eyes when the mask 20 is worn. It may be that the receivers 30 are permanently-formed indents or pockets in the eye coverage portion 22. For example, the indents or pockets can be positioned on the side of the mask that directly faces the eyes of the patient when worn. In use, two removable pods 50 are received in the mask 20, one within each of the receivers 30. Each eye coverage portion 22 can optionally include an eye cushion 32 and an eye cover 34. The eye cushion 32 is configured to surround the receiver 30 and provide padding on a portion of the mask 20 that abuts the users face when worn. The eye cover 34 is configured to cover the outside of the eye coverage portion 22. It may be that the mask 20 does not include an eye cover 34 and the eye coverage portion 22 is formed from a circular frame that is open when a detachable pod 50 is not in the receiver 30. The eye coverage portions 22 can be a substantially round shape as depicted in Figures 1-4 or can be an oval, elliptical, polygonal, angled or another non-round shape.

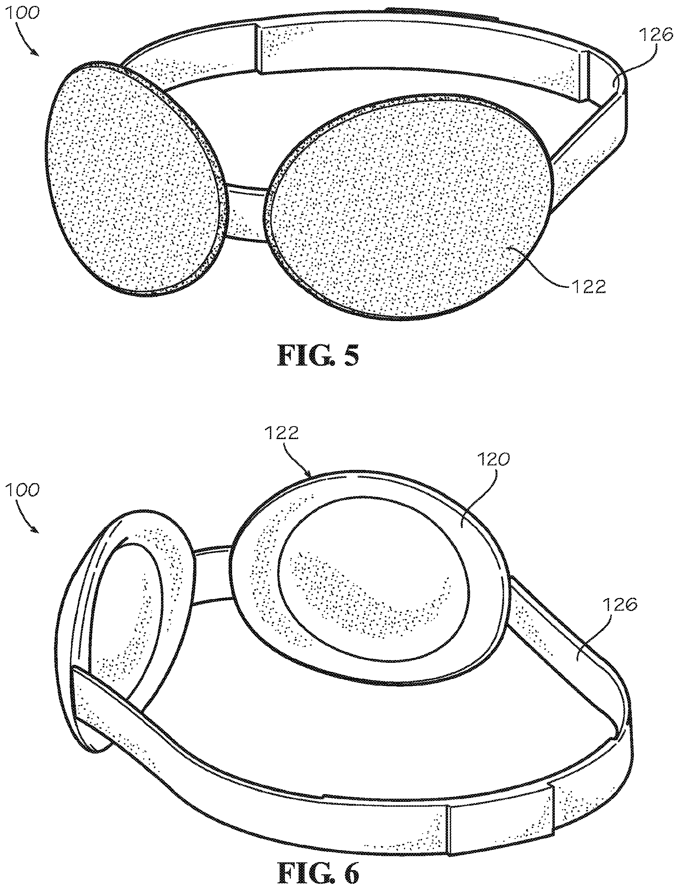

The eye coverage portions 22 can be connected by a nose bridge 24 and a securing strap 26. The nose bridge 24 can be formed from a flexible and/or elastic material that allows the nose bridge to fit the face shape of a variety of users. It may be that the nose bridge 24 is adjustable. As depicted, the securing strap 26 is formed from a strap extending between the eye coverage portions 22. The securing strap 26 can include an adjustment mechanism 40 that allows the user to adjust the length of the strap. Alternatively and as depicted in Figures 5 and 6, it may be that the securing strap 126 is formed from two straps, each coupled at a first end to an eye coverage portion 122. The second end of each securing strap 126 is configured to be removably coupled to the other securing strap. The attachment mechanism can include snaps, ties, hook-and-loop fasteners or other releasable attachment mechanisms. The nose bridge 24 and securing strap 26 are configured so that the eye mask 20 can be one size to fit all or most users. Alternatively, the eye mask 10 can be produced in different sizes. The securing strap can also include a comfort wrap (not pictured). Otherwise, it may be that the mask 20 can include dual supports to fit over the ears in place of the securing strap 26.

It may be that the mask portion 220 of the therapeutic eye mask system 200 is shaped similar to swimming goggles. The eye coverage portions 222 have a teardrop shape with the top and bottom having a curved shape and the side adjacent the nose bridge being straight so as to follow the angle of the nose. The detachable pods 250 have a generally oval shape and are angled relative to the nose bridge 224. The nose bridge 224 can include a padded portion 242 to provide comfort when worn. The eye covers 234 can be perforated for breathability. The securing strap 226 as depicted includes two adjustment mechanisms 240, one adjacent to each of the eye coverage portions 222.

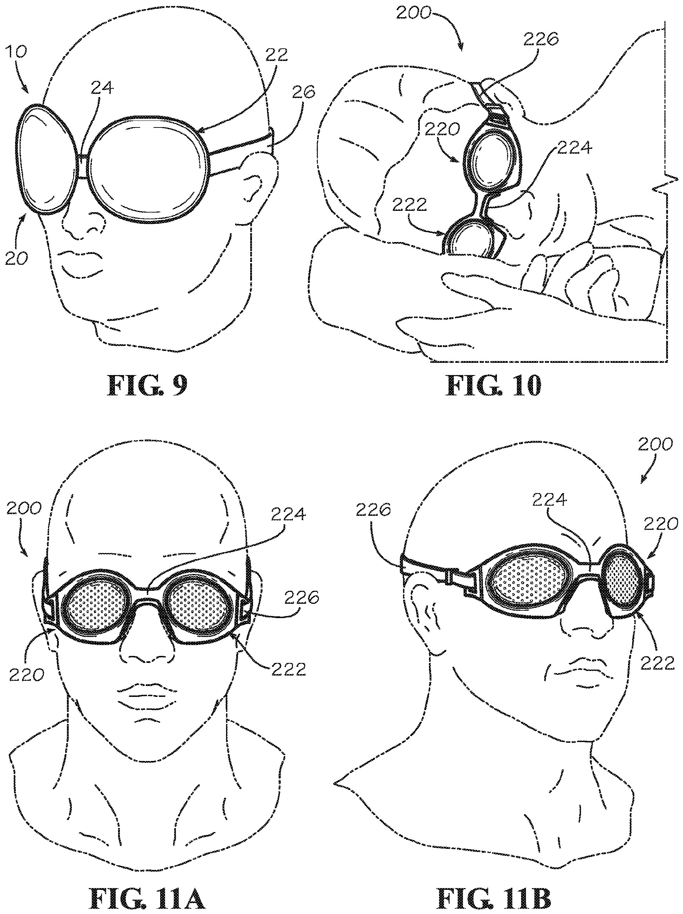

The mask 20, 220 portion of the therapeutic eye mask system 10, 200 is designed to fit securely to the patient's eye region with an eye coverage portion 22, 222 over each eye, the nose bridge 24, 224 positioned across the patient's nose and the securing strap 26, 226 extending around the back of the patient's head, as shown in Figures 9-11. The eye mask 20, 220 is designed such that it can be safe and comfortable to wear while sleeping, as shown in Figure 10. The secure fit can also prevent air from being blown into the eye while the mask is being worn, for example when worn in combination with a CPAP machine. It may be that the dual eye coverage portion design allows full conformance within the eye socket. The design also optionally allows heat and moist therapy to reach the sinuses to help relieve sinus pressure.

As discussed above, the eye coverage portions 22 of the mask 20 each include receivers configured to releasably secure a therapy pod 50. It may be that the pods 50 have a soft outer shell construction that is able to contain a fill material capable of delivering therapy treatment, for example, moist heat and cold treatments. The outer shell of the pods 50 can be formed of fabric, non-wovens or other natural or synthetic materials, and is preferably thermally and moisture transmissive, to allow heat and/or moisture to pass through the shell to and from the fill material. Example fill materials include hydrophilic zeolite granules or particles, and optionally silver or other antimicrobial treatments, and/or other materials. The fill material can be loosely contained and held within the pod 50 such that each pod will conform to the eye area of the patient when worn. Alternatively, a liquid or gel fill material can be used. The pods 50 can be designed for single use or can be washable and re-usable.

It may also be that the fill material contained within the pods comprises a synthetic porous crystalline granular aluminosilicate zeolite, for example, a hydrophilic natural or synthetic zeolite, also referred to as a molecular sieve material, or other substances with similar properties. The fill material may further comprise other inert additives and physical matrices without affecting the antimicrobial and hydrous efficacies of the fill. The hydrophilic zeolite granules or beads are configured to repeatedly absorb and release moisture without substantially changing shape or form. Optionally, the pods comprise a granular fill material such as activated alumina, silica gel, bentonite or hydrophilic zeolite or molecular sieve material. Otherwise, it may be that the pods comprise capsules or packets of non-granular material (e.g., gel, liquid), powder, or other materials. The pods or granules contained in the pods optionally also contain a metallic or other antimicrobial agent, such as for example a silver, copper, copper oxide, gold, magnesium oxide, aluminum oxide, titanium dioxide, zinc oxide, cobalt, nickel, zirconium, molybdenum, tin, lead and/or other metals, metal oxides, metal ions, metal particles or nanoparticles, and alloys, mixtures or combinations thereof deposited therein. For example, silver or another metal loading of the fill may be attained by the process of ion-exchange. In this process, a solution containing atomic silver or a composition of silver bathes or is passed through a bed of the fill granules. An ion-exchange column method may be performed in which an aqueous solution containing atomic silver or a composition of silver may be passed through a column bed of the fill granules, and the eluted solution may again be passed through the bed or may receive additional silver and then be again passed through the bed.

Various ion-exchange schedules as known in the art may be applied to produce retention of the silver or other metals in the fill material of the pods. For example, the final content by weight of an atomic silver or silver composition, or other metals or antimicrobial agents, may be as high as twenty percent of the final loaded fill granules. It may be that the loaded fill granules produced by ion-exchange will exhibit high retention of the silver or other metals even under subsequent exposure to fluids and microwave irradiation. The fill granules may comprise a blend of both metals loaded and unloaded (i.e., not containing metal) zeolite or other substance(s) retaining silver or other metals. The presence of the atomic silver or other metals preferably will not interfere with the useful properties of the fill granules such as the moisture desorption and adsorption properties which may be desirable in the use of the eye mask or compress system. The inherent hydrophilic nature of example forms of zeolite fill materials provides that substantial water content is available therein by absorption from the atmosphere. The water so absorbed may be sufficient for moist heat delivery applications, or may be supplemented by manually added water, for providing a microwave responsive water content of the eye mask or compress system. The compositions of silver or other metals used may include but are not limited to, metal compounds, and metal salts such as silver chloride and silver nitrate.

The presence of the silver or other metals within the fill granules optionally contained in the pods provides anti-microbial properties to the therapeutic eye mask system. The ion-exchange loaded fill granules will preferably retain the silver or other metals despite microwave heating as may be required in the use of the eye mask or compress system. Further, the retention of the silver or other metals within the fill granules provides assured antimicrobial performance in a reusable and potentially washable, if so desired, moist heat therapy compress. Otherwise it may be that the silver or other metals are incorporated into the cover material of the pods, the eye coverage portions, and/or other portions of the eye mask system, in addition to or instead of the fill granules. Alternatively, one or more non-metal antimicrobial materials and/or medications may optionally be incorporated into the fill material, the pods, the eye coverage portions, and/or other portions of the eye mask system.

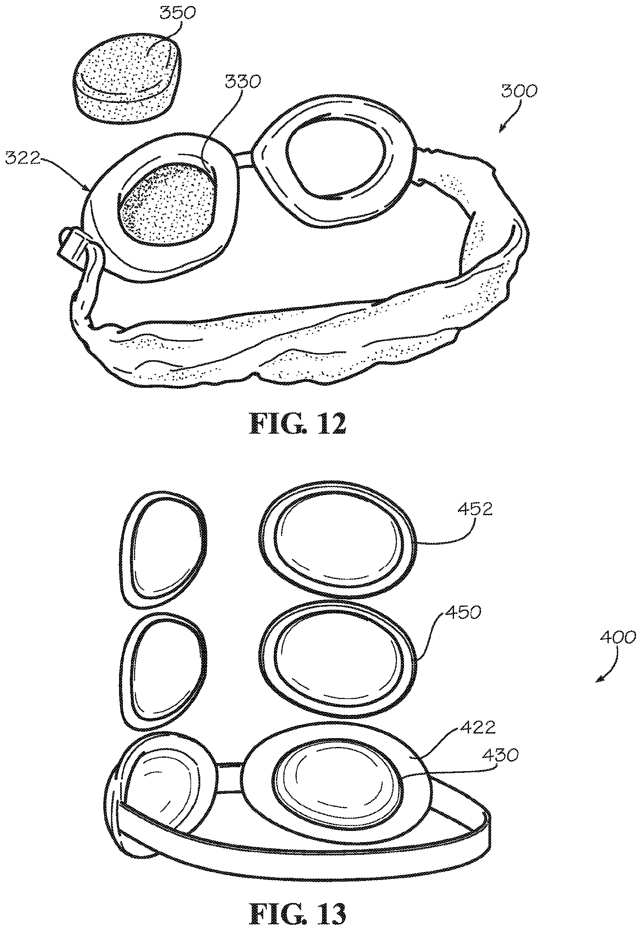

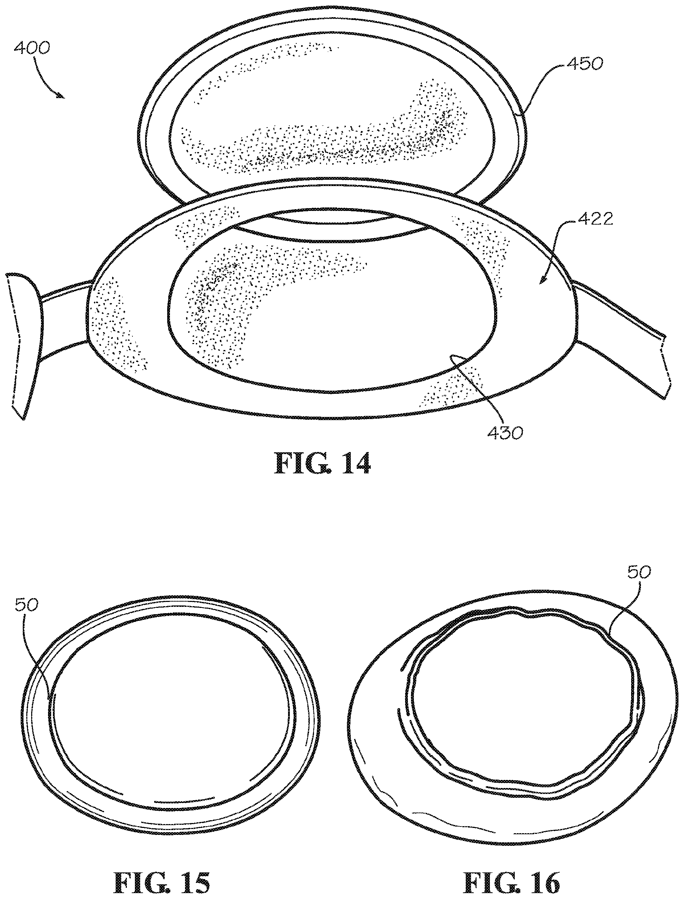

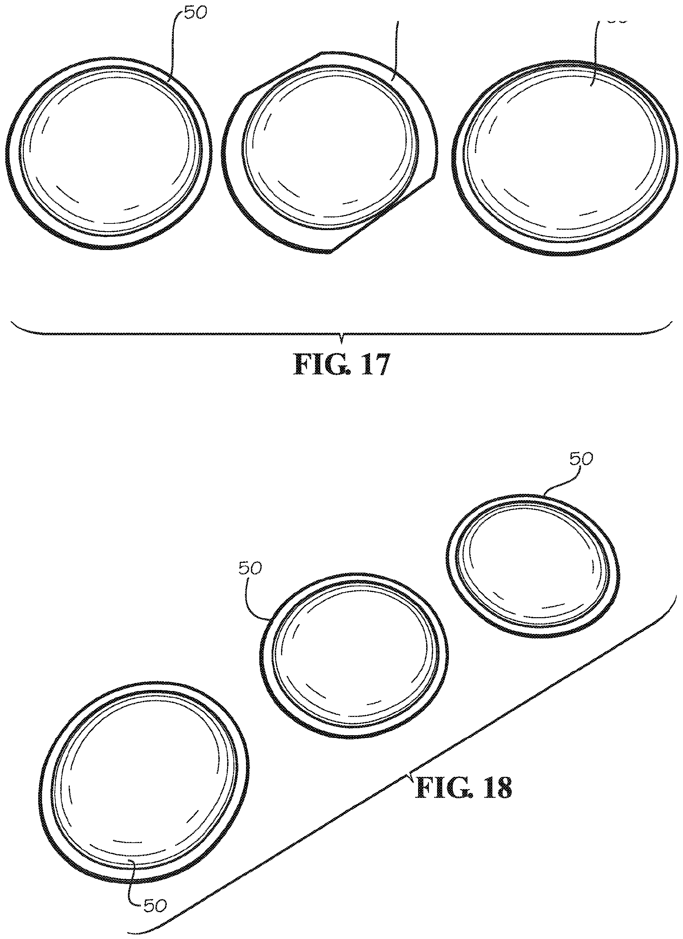

In example embodiments of the therapeutic eye mask system 300, the pods 350 are push-fitted into the receivers 330 in each eye coverage portion 322, as shown in Figure 12. The pods 350 can be detachably secured within the receivers 330 through the use of an attachment mechanism, for example, a hook-and-loop fastener material secured to both the pods and the receivers, by friction fit, or otherwise. Alternatively, the pods 350 are secured with a positive lock, clip, or a rigid snap. It may be that the pods 450 can be slid into the receivers 430 through an opening in the top of the eye coverage portion 422, as shown in Figure 14. The pods 50 may be round so that they are self-orienting when attached or inserted into the mask, or can be oval, elliptical, polygonal, angled or another non-round shape, as shown in Figure 17. The pods can be provided in one or more different colors, for example, color coded based on their function. For example, a pod for moist heat treatment may be red and a pod for cool treatment may be blue. The pods 50 can also be provided in different sizes, as shown in Figure 18. It may be that the material and configuration of the mask form a seal against the wearer's face surrounding the eye area to prevent external airflow from drying the wearer's eyes or drying the pods or medication components positioned within the mask during use.

In use, the entire mask with the pods attached can be heated or cooled prior to use to provide therapy. The mask can be placed in the microwave to be heated or in the freezer to be cooled. Alternatively, the pods alone can be heated or cooled detached from the mask, then placed in the mask when they reach the desired temperature for treatment.

Additionally, various medicaments can be applied and used with respect to the pods. For example, one or more ophthalmic medications can be infused or injected into the formulation in the pods. Examples of medicants or therapeutic materials capable of delivery using the therapeutic device include a jojoba formulation for treatment of the symptoms of dry eye, steroids such as clobetasol propionate, betamethasone dipropionate, amcinonide or loteprednol etabonate for treatments of diseases of the eyelid, such as chatazion, blepharitis or meibomian gland dysfunction. Medicants may also comprise a dietary or nutritional supplement composition comprising an effective amount of omega-3 fatty acids for treatment of dry eye or meibomianitis. Medicants may also comprise tetracycline, corticosteroids, androgens or androgen analogues. The medicant can also comprise a topical treatment to elevate the side effects of chemotherapy, including eyelash loss.

The therapeutic eye mask system 400 may be combined with a heat-transmissive pad or lid scrub or disc 452 that is applied to the skin surface on the eyelid and around the eye, as shown in Figure 13. It may be that the pad or disc is constructed of a non-woven material and optionally a material that can be RF or thermally sealed to hold an antibacterial or other medication. The pad or disc 452 can be impregnated with medication and can be effective in either a moist or dry condition. The antibacterial medication can include, for example, liposomes and/or microspheres. The pad or disc 452 can be removably secured to the compress for single use or multiple uses. The pad can also be impregnated with materials to improve the aesthetics of the eye, like Vitamin E.

It may be that the medication pad or disc 453 is moist-heat-transmissive, and application of moist-heat activates the release of the impregnated medication from the pad 452 onto the skin surface of the eyelid or other tissue in or around the user's eyes. In example forms, the pad or disc is constructed to prevent the impregnated medication from passing back into the compress during application. For example, a one-way sheet barrier material can be placed between the pad or disc and the compress to prevent any antibacterial medication from the disc from entering the compress.

It may also be that the medication pad or disc 452 can have a round or oval disc shape with appropriate size and shape to be placed over a single eyelid or attached to a single eye coverage portion 422. In use, two pads or discs can be used, one attached to each eye coverage portion 422. A plurality of discs can be stored within a container containing antibacterial medication fluid, so as to pre-moisten the discs during storage. In an exemplary manner of use, two discs are removed from the container and one is placed over each eye of a patient while the patient is lying down and the mask is placed over the discs so that the discs are held in between the eye and the eye coverage portion. Alternatively, the disc 452 may be attached to the detachable pod 450, such that when the eye mask or compress is worn, the disc rests in between the eyelid and the detachable pod. Otherwise, it may be that the disc 452 is placed in the receiver 430 with the detachable pod 450.

Otherwise, it may be that the medicated disc is used as an eyelid cleaning or treatment wipe. The medicated disc is impregnated with a dry specialized formula that is activated by the heat and moisture from the mask. The medication assists in preparing debris in the eye for removal. The disc can then be removed from the mask and used to scrub or wipe the eye area, removing the debris. The disc can be formed of a scrim or non-woven material that accepts dry impregnation of specialized formulas or medication. Ideally, the medicated disc allows the passage of moisture and heat. The medicated disc can remove oil debris and pollen from the eyelids and enhance the moisture of the skin around the eye. The disc can also protect the mask itself from make-up or other contaminants.

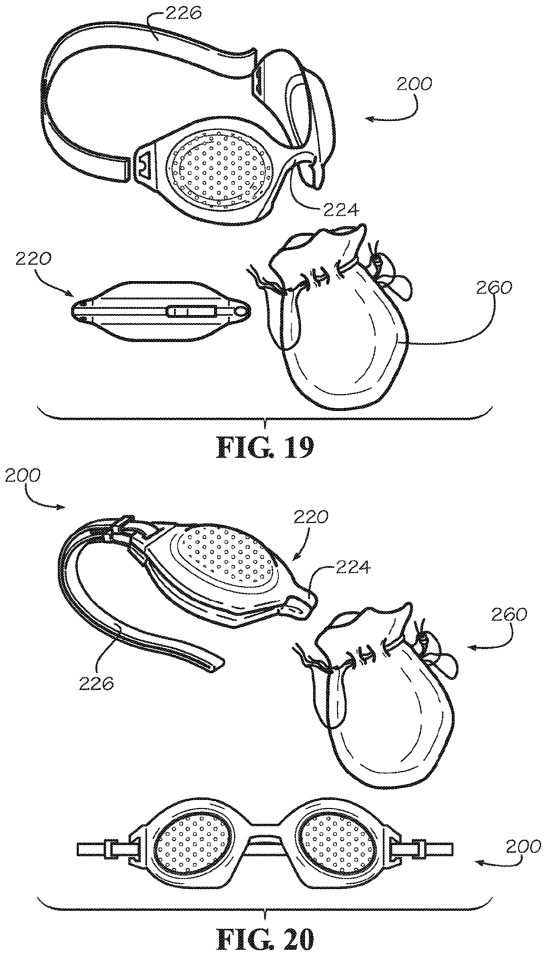

The therapeutic eye mask system 200 can also include a storage bag 260, as shown in Figures 19 and 20. In the depicted embodiment the mask portion 220 is configured to fold at the flexible nose bridge 224 to fit within the storage bag 260. The securing strap 226 can be detached from the mask 220 for storage, as shown in Figure 19, or the mask 220 can be stored with the securing strap 226 attached, as shown in Figure 20.

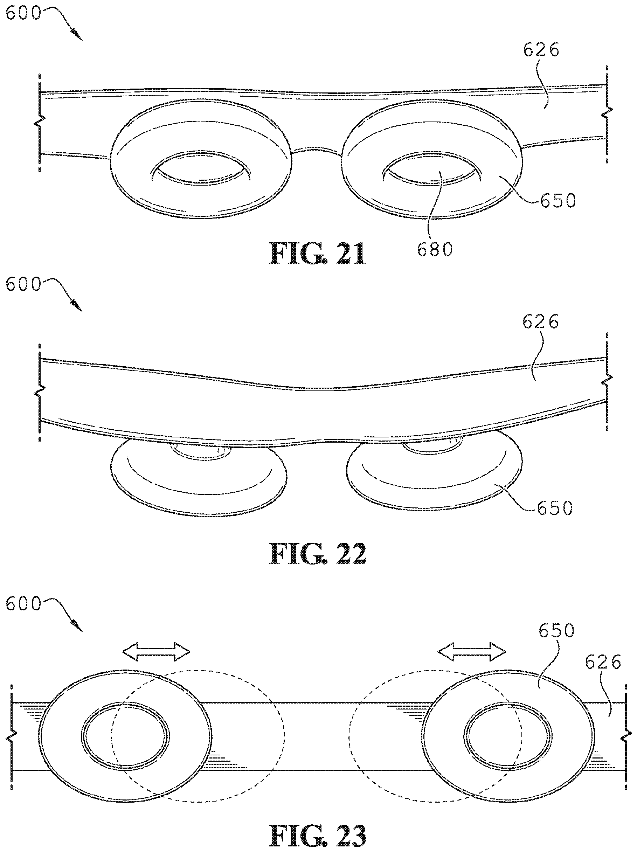

Figures 21-24 show a therapeutic eye mask system 600 according to an example embodiment of the invention. The eye mask system 600 includes a mask portion or strap 626 and one or more pods 650 that are detachably secured to the strap. In this embodiment, the pods 650 are attached directly to the strap 626 without the need for a receiver as in previous examples. The strap 626 is positioned around the user's head to hold the pods 650 in contact with the eye area of the user. The pods 650 are releasably attached to the strap 626 using an attachment mechanism such as snaps, ties, hook-and-loop fasteners or other releasable attachment mechanisms. The pods 650 are attached directly to the strap 626 such that the back side of each pod directly contacts the front of the strap. The front of each pod 650 faces away from the strap 626 and is configured for contact with the eye area of the patient. In example embodiments, the pods 650 can be attached at a plurality of points along the strap 626, as shown in Figure 23, such that the user can adjust the pods to fit their unique face shape. The pods 650 can also be used independently of the strap 626.

The pods 650 include a divot, recess, depression, or other void 680 configured to be positioned over the wearer's corneas when the mask is worn. As explained above, high temperatures may be unsafe for the cornea and the divot or recess 580 positioned over and around the cornea can help prevent the application of excess heat on and around the cornea, preventing conditions such as corneal warping, while allowing heat therapy to reach the eyelid and surrounding eye area.

As in previous examples, the pods 650 comprise an outer shell 652 that surrounds and contains a fill material 654. The pod shell 652 is formed from a flexible material such as a fabric. In example embodiments, the pod shell 652 is formed, in whole or in part, from a material with antimicrobial properties. The pod shell 652 material can incorporate an antimicrobial substance, such as silver or other antimicrobial metals. In example embodiments, silver salts or particles are attached to the pod shell fabric. In other embodiments, silver salts or particles are incorporated into fabric fibers such as polyester fibers. The silver particles are encapsulated by the plastic which protects them during manufacturing and use of the mask. The silver impregnated yarn is woven into the fabric of the pod shell 652. The silver impregnated yarn helps ensure that the antimicrobial silver particles are distributed throughout the pod shell 652. The mask can maintain its antimicrobial properties through repeated uses and laundering. The antimicrobial material is configured to kill bacteria in and around the user's eye by contact. In example embodiments, the antimicrobial material will kill 99% of bacteria after 4 hours of contact. In example embodiments, the section of the pod shell 652 that contacts the user's face includes the antimicrobial fibers. In other embodiments, the entire pod shell 652 is formed from a material incorporating antimicrobial fibers. In example embodiments, other components of the mask, such as the strap, are also formed from an antimicrobial material as described above.

The fill material 654 generally comprises a plurality of fill beads or granules. The fill material 654 is contained by the pod shell 652 such that it remains within the shell and does not fall out. The fill material 654 is generally able to move within the pod shell 652 such that the shape of the pod 650 will conform to the face of the user. In example embodiments, the fill beads or granules 654 can be formed from a resilient, deformable material, such as silicon. The resilient, deformable fill beads contribute to the comfort of the user.

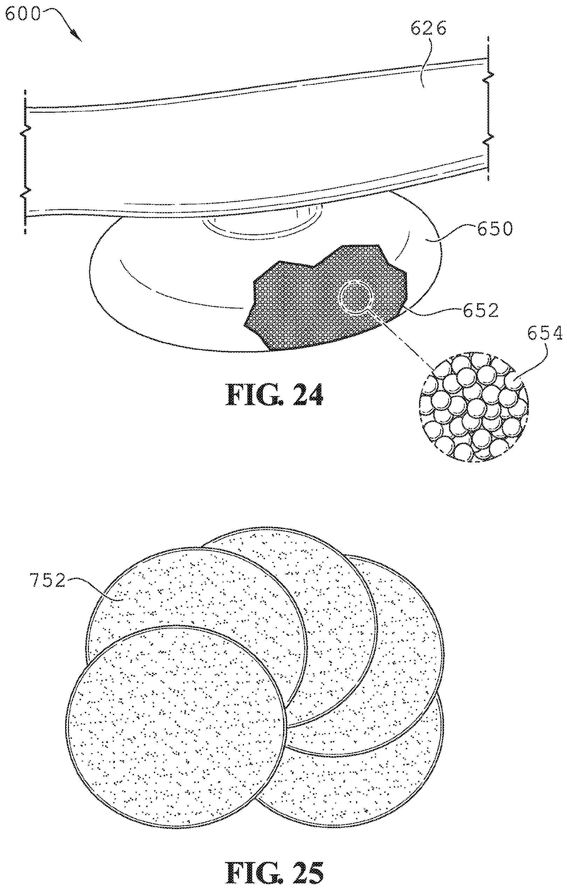

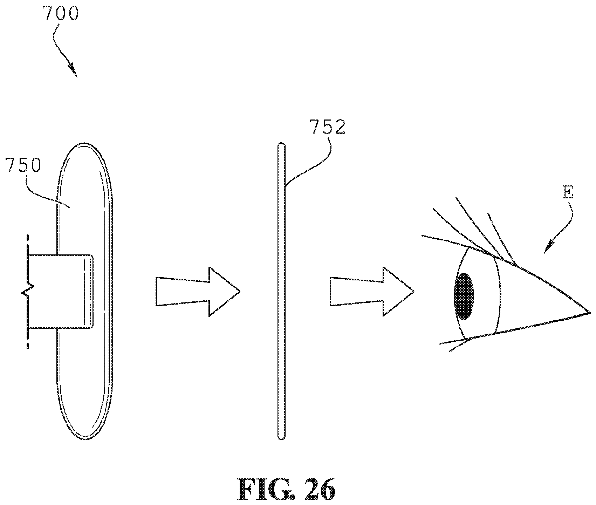

Figures 25-26 show a therapeutic eye mask system 700 according to another example embodiment of the invention. The mask system 700 includes a therapeutic mask similar to those described above. The mask system 700 also includes a treatment sheet 752 that can be placed between the eye E of the user and the pod 750 or eye coverage portion. The treatment sheet 752 is generally impregnated with a unique therapeutic formula designed for a specific treatment of the eye area E of a patient. In example embodiments, the treatment sheet 752 is heat-transmissive, such that when the treatment sheet is heated or exposed to a heat source, the impregnated medication is released from the sheet and is able to travel to the eye area of the patient. The treatment sheets can be dry when impregnated with medication. These treatment sheets or pads can be used with a dry heat source. These treatment sheets 752 do not have to be stored in an air-tight container to prevent drying out. These treatment sheets also can be used without having to wet the sheet prior to use.

In example embodiments, the treatment sheets 752 can be a heat-transmissive pad or lid scrub or disc that is applied to the skin surface on the eyelid and around the eye E. The pad or disc 752 can be constructed of non-woven material and optionally a material that can be RF or thermally sealed to hold an antibacterial or other medication. The pads or sheets 752 can be formed from any material that accepts treatment materials and holds them in a dry state until activated by moisture and/or heat. The pad or disc can be impregnated with medication and can be effective in either a moist or dry condition.

In example embodiments, the medication pad or disc is moist-heat-transmissive, and application of moist-heat source activates the release of the impregnated medication onto the skin surface of the eyelid or other tissue in or around the user's eyes. In example forms, the pad or disc is constructed to prevent the impregnated medication from passing through the back of the disc away from the treatment area. For example, a one-way sheet barrier material can be placed between the pad or disk and the compress to prevent any antibacterial medication from the disk from entering the compress.

In example embodiments, the treatment pad or disk 752 can have a round or oval shape, as shown in Figure 25. The treatment disk 752 is generally a size and shape to be placed on over a single eyelid. In use, two pads or disks can be used, one treats each eye. In alternate embodiments, the sheets can be shaped to cover the entire eye treatment area. A plurality of disks can be stored within a container containing antibacterial medication fluid, so as to pre-moisten the disks during storage. In other embodiments, a medication can be sprayed on the treatment pads prior to use.

In an exemplary manner of use, two disks are removed from the container and one is placed over each eye of a patient while the patient is lying down. The disk or sheet is exposed to a heat source, causing the impregnated medication to be released towards the eye. Alternatively, the disk may be detachably or permanently attached to a reusable or disposable compress delivering dry or moist heat, such that when the eye mask or compress is worn, the disk rests in between the eyelid and the detachable pod. The pad or sheet can include a means for attaching the pad to a compress. Attachment means can include an adhesive material or a fastening device such as a hook and loop fastener. In alternate embodiments, the pad or sheet can be activated by another device producing dry or moist heat, such as a humidifier, a steam or water vapor generator, a heating pad, etc.

Examples of medicants or therapeutic materials capable of delivery using the therapeutic device according to example forms of the invention include a jojoba formulation for treatment of the symptoms of dry eye, steroids such as clobetasol propionate, betamethasone dipropionate, amcinonide or loteprednol etabonate for treatments of diseases of the eyelid, such as chatazion, blepharitis or meibomian gland dysfunction. The medicant can include honey, for example, manuka honey. The medicant or medication can include natural oils including coconut or tea tree oils for the treatment of conditions including Blepharitis. The medicant can be an antibacterial medication including, for example, liposomes and/or microspheres. Medicants may also comprise a dietary or nutritional supplement composition comprising an effective amount of omega-3 fatty acids for treatment of dry eye or meibomianitis. Medicants may also comprise tetracycline, corticosteroids, androgens or androgen analogues. The medicant may also comprise a topical treatment to elevate the side effects of chemotherapy, including eyelash loss. The medicant can include menthol configured to stimulate lacrimoation via activation of cold-sensitive primary afferent neurons in the cornea. Repeated use of menthol can induce persistent increases in tear fluid volume and tear film stability in dry eye patients.

In further embodiments, the therapeutic device takes the form of a hygienic cleaning sheet or pad, including a heat-transmissive substrate configured for cleaning away makeup, debris, oils, contaminants or other materials from a user's eyelids, eyelashes, and surrounding tissue. The sheet or pad may be utilized for hygienic cleansing before, during and/or after application of heat. The substrate is optionally dry coated with one or more natural oils or other hygienic cleansing materials such as for example coconut or tea tree oils, manuka or other honey, menthol, and/or vitamin E. The natural oils or other hygienic cleansing materials optionally provide antibacterial or antimicrobial treatment. In example embodiments, the natural oils or other hygienic cleansing materials are dry coated onto / into the substrate, and configured for release from the substrate toward and onto the eyelids and surrounding tissue or other body parts of a human or animal user, upon application of heat (including moist heat) to the therapeutic device and/or the targeted tissue or body part(s). The natural oils or other hygienic cleansing materials are preferably activated to therapeutically effective levels by application of heat (including moist heat) at safe and comfortable temperatures and moisture levels.

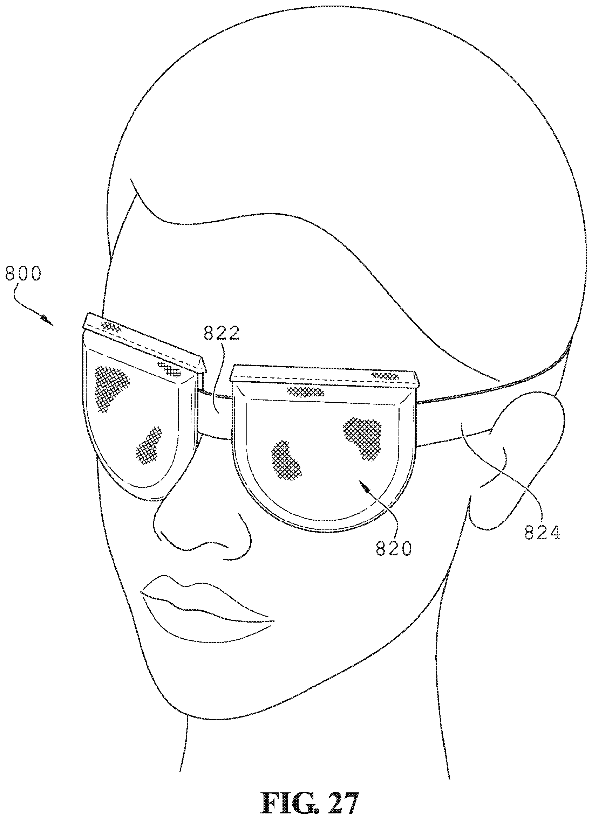

Figure 27 shows a therapeutic eye mask or compress 800 according to another comparative example. It may be that the mask 800 includes at least one, and as depicted comprises two separate eye coverage portions 820, each configured to be positioned over the user's eyes when the mask is worn and deliver moist heat therapy. The eye coverage portions are connected by a flexible nose bridge 822 and a securing strap or head band 824, as shown in Figure 27. The nose bridge 822 and/or securing strap 824 may comprise a flexible elastic material or strip, and optionally also comprise one or more attachment or the like, to allow attachment and optionally also comprise one or more attachments or connection elements such as hook-and-loop attachment material, snaps, clips, buttons or the like to allow attachment and optionally adjustment of the fit around the user's head. The mask 800 is configured to be worn on the head of a human or animal patient with one of the eye coverage portions 820 against or adjacent over the eyes of the user, as shown in Figure 27. Otherwise, it may be that a mask or compress having a single eye coverage potion is provided, allowing the user to treat one eye at a time, leaving the other eye uncovered.

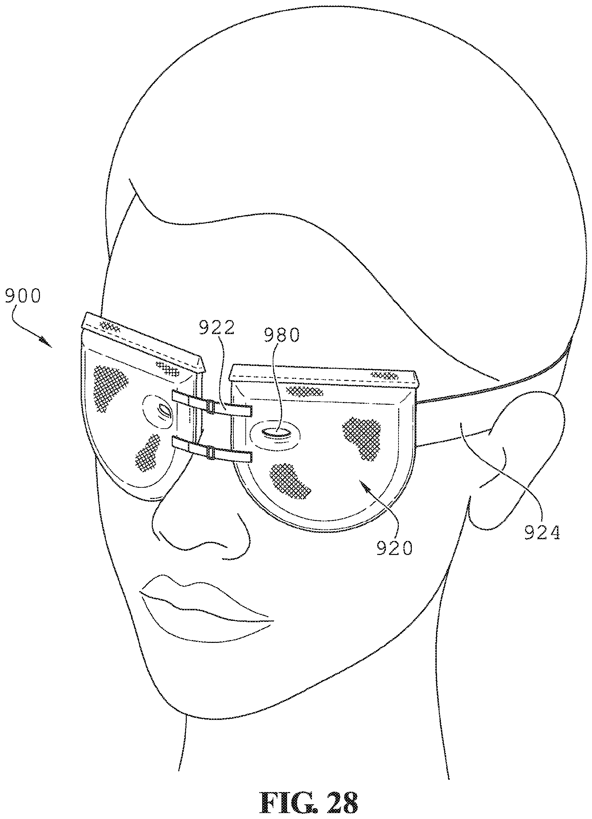

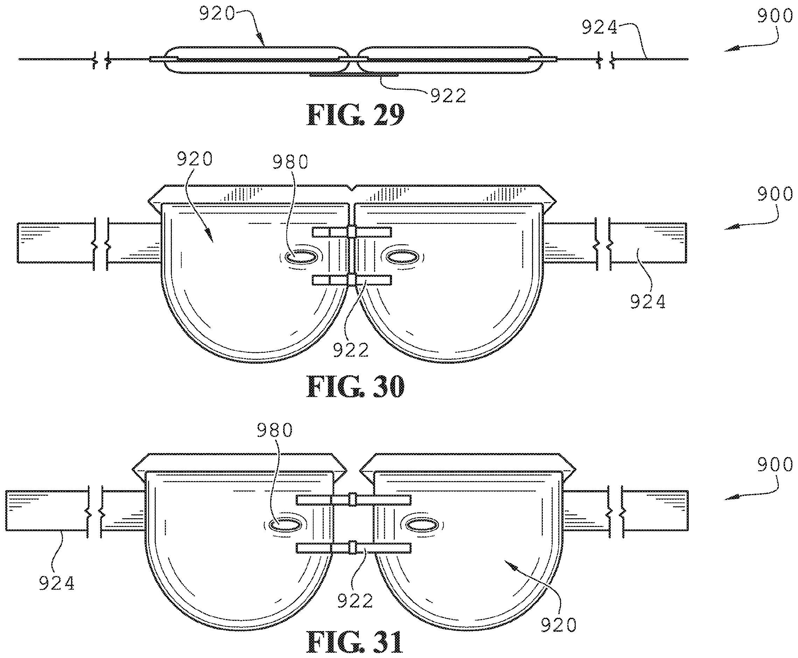

Figures 28-31 show a mask 900 according to another example embodiment of the present invention. In the depicted embodiment, each eye coverage portion 920 includes an opening, recess, depression, slit or another void 980 configured to be positioned over the wearer's corneas when the mask is worn. Temperatures over 39.5° Celsius may be unsafe for the cornea. The openings or voids 980 positioned over and around the cornea can help prevent the application of excess heat on and around the cornea, preventing conditions such as corneal warping, while allowing moist heat therapy to reach the eyelid and surrounding eye area. In example embodiments, the cornea voids 980 are around 15 mm wide. In example embodiments, the voids 980 can be recessed in the eye coverage portion 920 or detachable pods or alternatively can be slits or openings extending through the entire thickness of the eye coverage portion and detachable pods whereby the user can see through the openings when worn. In alternate embodiments, the eye coverage portions or detachable pods can include a heat insulating material applied over one or more portions of the eye covers configured to be positioned over the corneas when in use. For example, an insulating shield can be attached to the central regions of each eye cover 920 or detachable pod. The insulating shield is configured to vault the cornea and prevent the application of excess heat on or around the corneas. This embodiment can further include an adjustable, flexible nose bridge 922. The adjustable nose bridge 922 allows the user to adjust the spacing distance between eye coverage portions 920 and/or between the voids in each eye cover to accommodate varying distances between human eyes from one individual to the next. The average pupillary distance for a human is around 57 mm to 65 mm. In example embodiments, the adjustable nose bridge 922 can be changed to accommodate a distance between cornea areas ranging from about 45 millimeters to about 74 millimeters. This range is sufficient to accommodate the eye placement of the majority of adult and/or child male and female humans. In example embodiments, one or more adjustable or expandable straps or bands are provided between the eye covers to allow adjustment of the relative positions or spacing of the eye covers. Alternatively, the eye covers can be detachably mounted to the retention strap, for example with hook-and-loop fastener material, to allow for repositioning of the eye covers on the strap to vary the spacing and position of the eye covers.

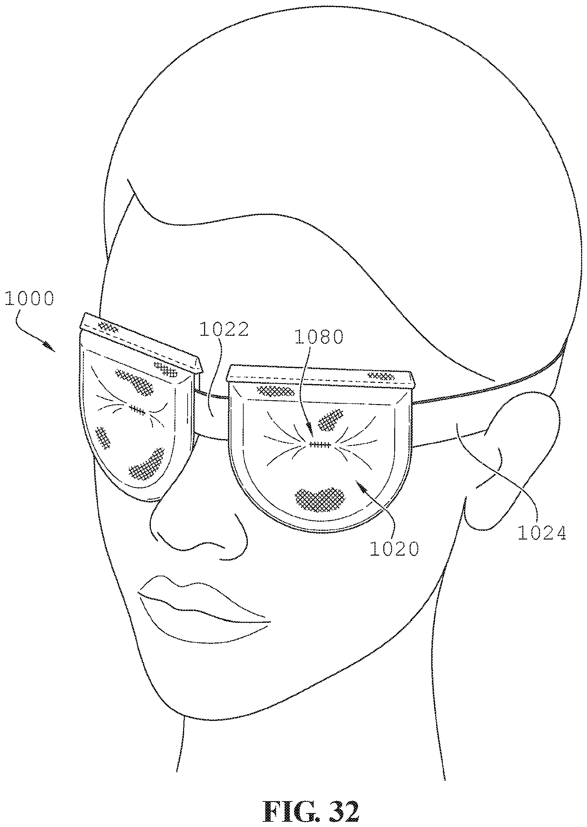

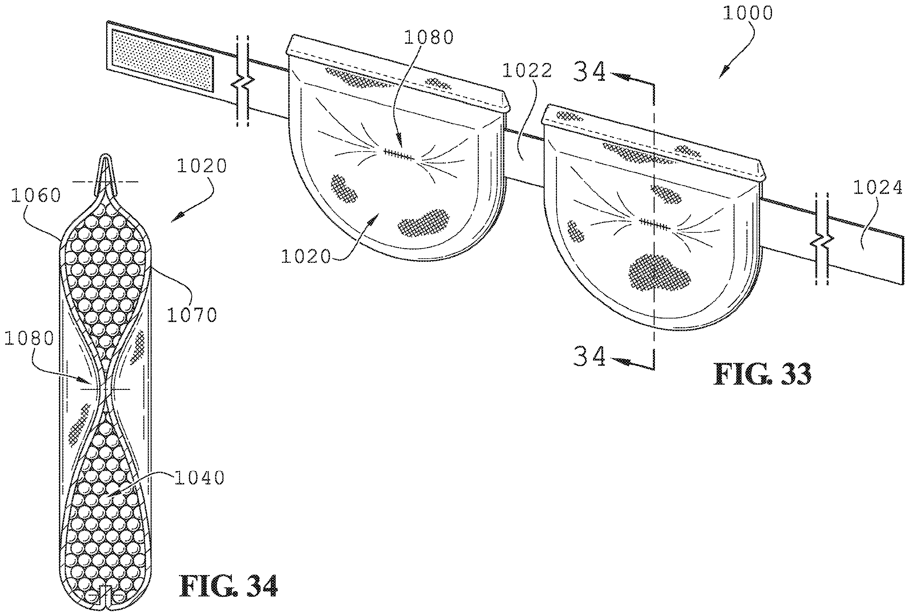

Figures 32-34 show a therapeutic eye mask or compress 1000 according to another example embodiment of the present invention. The therapeutic eye mask 1000 generally includes at least one, and in the depicted embodiment two separate eye coverage portions 1020, each configured to deliver moist heat therapy to the eye regions of the user, connected by a nose bridge 1022 and a securing strap 1024 for placement around the user's head to hold the compress 1000 in place, as shown in Figure 32-33. In example embodiments, the nose bridge 1022 and/or the securing strap 1024 may comprise a flexible elastic material or strip, and optionally also comprise one or more attachment or connection elements such as hook-and-loop attachment material, snaps, clips, buttons or the like, to allow attachment and optionally adjustment of the fit around the user's head. One or more size-adjustment connections are optionally provided to allow the user to fit the compress 1000 to their head and eye regions. The mask 1000 is configured to be worn on the head of a human or animal patient with one of the eye coverage portions 1020 positioned over a respective eye of the patient. In example embodiments, the securing strap 1024 is positioned around the user's head to hold the eye coverage portions 1020 against or adjacent over the eyes of the user, as shown in Figure 32. In alternate embodiments, a mask or compress having a single eye coverage portion is provided, allowing the user to treat one eye at a time, leaving the other eye uncovered, or a larger mask configured to cover both eyes may be provided.

Each eye coverage portion 1020 has an inner front face or application side 1070, configured to rest against the face of the patient (the inner or patient treatment side of the compress), and an outer back face or distal side 1060 opposite the front face (the outer or distal side of the compress). In example embodiments, the inner or front face 1070 of the eye coverage portion 1020 has a different appearance and/or texture than the outer or back side 1060, to allow a user to readily differentiate which side of the compress is the patient treatment side to be applied over and against the patient's eye region. For example, the inner or front side 1070 may comprise a smoother or softer material without coloration or printing, whereas the outer or back side 1060 may comprise a more durable or rougher textured material having branding text or logos, or other coloration or indicia imprinted or otherwise applied thereon. Alternatively, instructional information may be imprinted or otherwise applied on one or both sides (for example, "this side toward you" printed on the inner or front side).

As depicted, each eye coverage portion 1020 also includes a recess or depression 1080 created by a segment of stitching, suture or other means of attachment between the inner face 1070 and the outer face 1060, forming a pinched or retracted central region in each eye coverage portion. The recess 1080 is configured to be positioned over the center of the wearer's eye to protect the wearer's corneas from excess heat and/or to provide improved comfort when the mask is worn. Temperatures over 39.5° Celsius may be unsafe for the cornea. The recesses 1080 are positioned over and around the cornea to provide a localized non-contact or lesser contact area to help prevent the application of excess heat on and around the cornea, preventing conditions such as corneal warping, while allowing moist heat therapy to reach the eyelid and surrounding eye area. In example embodiments, the recess or depression 1080 is formed in at least the inner or front side 1070 of the eye coverage portion(s), and optionally in both the inner side 1070 and the outer side 1060 as depicted. In example embodiments, the stitched segments 1080 are about 5-15 mm wide, and preferably about 8-12 mm wide, for example about 10 mm wide. In example embodiments, the recessed regions 1080 are formed by sewing a thread through both the inner and outer face panels 1070, 1060, or alternatively by adhesive attachment, thermal bonding, staple, clip or other attachment or coupling means. In example embodiments, the stitched recesses 1080 can be formed in the eye coverage portions 1020 as depicted or in detachable pods as described elsewhere herein.

As in previous examples and embodiments, the eye coverage portions 1020 comprise an outer fabric shell that surrounds and contains a loose granular or particulate fill material 1040. The shell is formed from a flexible material such as a knitted or woven fabric. In example embodiments, the shell is formed, in whole or in part, from a material with antimicrobial properties. For example, the shell material can incorporate an antimicrobial substance, such as silver or other antimicrobial metals. In example embodiments, silver salts, fibers or particles are attached to or embedded or woven within the eye coverage shell fabric. In other embodiments, silver salts or particles are incorporated into fabric fibers such as polyester fibers. The silver particles are optionally encapsulated by the fiber material which protects them during manufacturing and use of the mask. In example embodiments a silver impregnated yarn is woven into the fabric of the shell. The silver impregnated yarn helps ensure that the antimicrobial silver particles are distributed throughout the eye coverage portion shell. The mask can maintain its antimicrobial properties through repeated uses and laundering. The antimicrobial material may be configured to kill bacteria and pathogens in and around the user's eye by contact. In example embodiments, the antimicrobial material will kill 99% of bacteria after 4 hours of contact. In example embodiments, the section of eye coverage portion 1020 that contacts the user's face includes the antimicrobial fibers. In some embodiments, the entire eye coverage portion 1020 is formed from a material incorporating antimicrobial fibers. In particular example embodiments, the shell fabric comprises 5-15% elastic fabric, 25-35% material with antimicrobial properties, and 50-70% regular fiber, including but not limited to cotton, polyester, wool, silk, and flax, and/or combinations thereof. In further embodiments, the shell fabric is formed from about 10% elastic fabric, about 30% material with antimicrobial properties, and about 60% regular fiber. In example embodiments, the material incorporating antimicrobial properties may comprise a PurThread^{®} material and the elastic material may comprise Lyrcra^{®}. In example embodiments, other components of the mask, such as the strap, are also formed from an antimicrobial material as described above.

The fill material 1040 generally comprises a plurality of fill beads or granules. The fill material 1040 is contained within each eye coverage portion 1020 such that it remains within the shell and does not fall out. The fill material 1020 is generally able to flow or move within the eye coverage portions such that the shape of the coverage portion 1020 will conform to the face of the user. In example embodiments, the fill beads or granules 1020 can be formed from a resilient, deformable material, such as silicon. The resilient, deformable fill beads contribute to the comfort of the user. In example embodiments, the fill material 1040 contained within the eye coverage portion 1020 comprises a synthetic porous crystalline granular aluminosilicate zeolite, for example, a hydrophilic natural or synthetic zeolite, as previously disclosed herein.

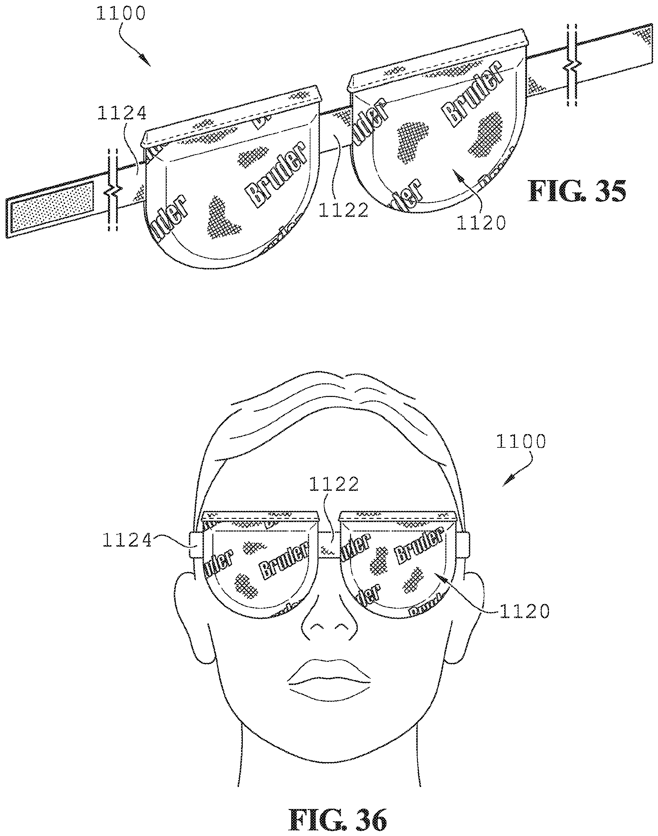

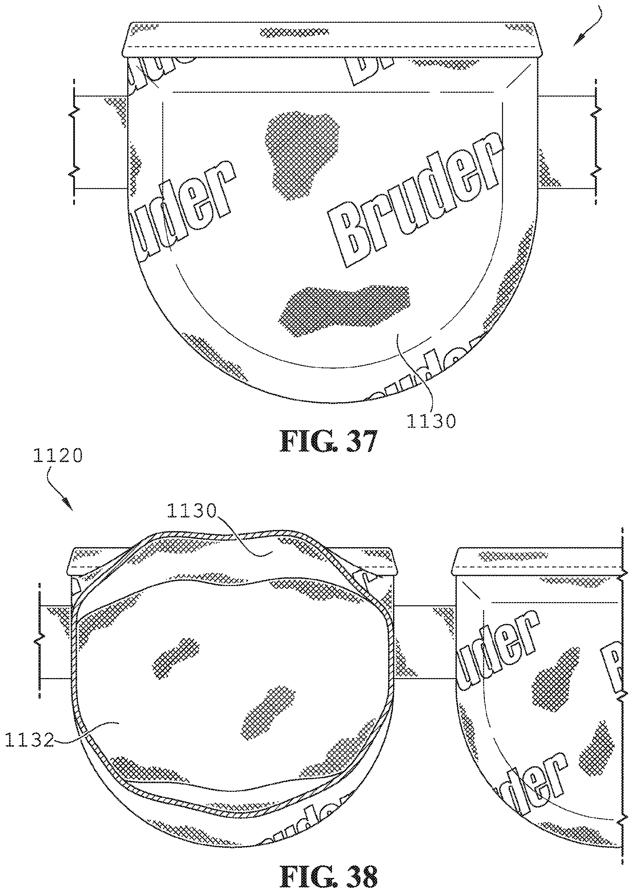

Figures 35-42 show a therapeutic eye mask or compress according to another comparative example. The therapeutic eye mask 1100 generally includes at least one, and as depicted includes two separate eye coverage portions 1120, each configured to deliver moist heat therapy, connected by a nose bridge 1122 and a securing strap 1124, as shown in Figure 35. The nose bridge 1122 and/or the securing strap 1124 may comprise a flexible elastic material or strip, and optionally also comprise one or more attachment or connection elements such as hook-and-loop attachment material, snaps, clips, buttons or the like, to allow attachment and optionally adjustment of the fit around the user's head. The mask 1100 is configured to be worn on the head of a human or animal patient with one of the eye coverage portions 1120 positioned over each eye of the patient. It may be that the securing strap 1124 is positioned around the user's head to hold the eye coverage portions 1120 against or adjacent over the eyes of the user, as shown in Figure 36. It may also be that a mask or compress having a single eye coverage portion is provided, allowing the user to treat one eye at a time, leaving the other eye uncovered. Each eye coverage portion 1120 has a front side or end 1126, configured to rest against the face of the patient (the inner or patient treatment side of the compress), and a back side or end 1128 opposite the front side or end (the outer or distal side of the compress). Figure 37 shows a detailed view of the back side 1128 of an eye coverage portion 1120. It may be that the inner or front side 1126 of the eye coverage portion 1120 has a different appearance and/or texture than the outer or back side 1128, to allow a user to readily differentiate which side of the compress is the patient treatment side to be applied over the patient's eye region. For example, the inner or front side may comprise a smoother or softer material without coloration or printing, whereas the outer or back side may comprise a more durable or rougher textured material having branding text or logos, or other coloration or indicia imprinted or otherwise applied thereon. Alternatively, instructional information may be imprinted or otherwise applied on one or both sides (for example, "this side toward you" printed on the inner or front side). Each eye coverage portion 1120 includes a moisture barrier material 1134, positioned towards the back or outer side 1128 of the eye coverage portion, configured to prevent moisture from escaping from the back or outer side of the eye coverage portion, thereby directing moist heat therapy toward the front or inner side for application to the eye region of the patient (i.e., in a patient-treatment direction).

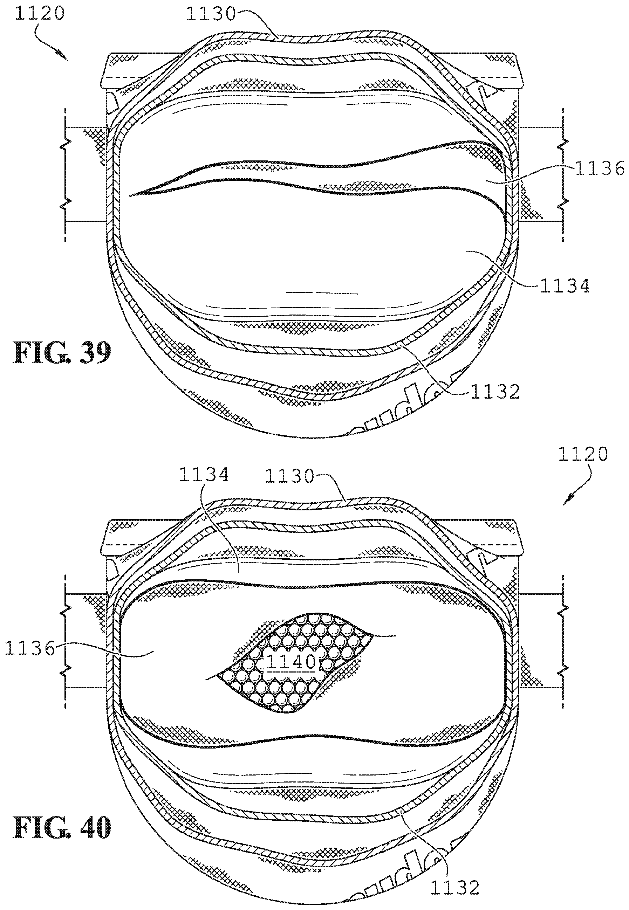

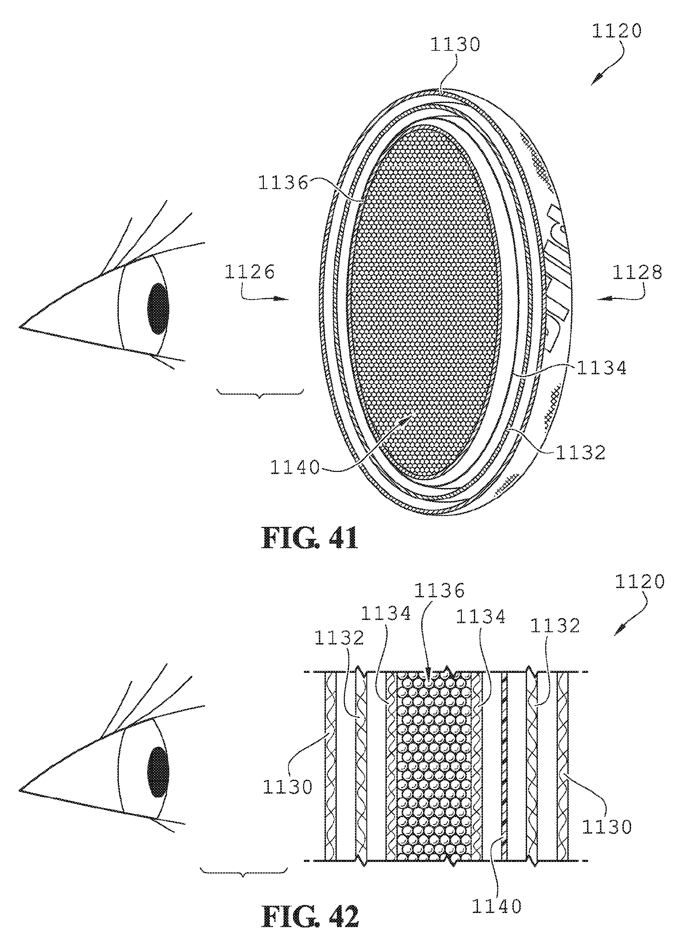

The eye coverage portions 1120 may be independent structures such that each can independently conform to the respective eye region of the patient. For example, as shown in greater detail in Figure 37, the eye coverage portion 1120 has a radially curved lower or bottom profile, and a generally rectangular upper or top profile, defining a generally U-shaped configuration. Optionally, an upper attachment strip is stitched along the top edge of the eye coverage portion. Alternatively, the eye coverage portions can be combined in a single panel or mask format configured to extend over both eyes. As depicted , each eye cover portion 1120 is formed from a series of layers or lamina. Figures 38-40 show successive layers being cut away and retracted to show the series of layers or lamina, and Figure 41 shows a cross--sectioned view of a portion of an eye cover with the successive layers or lamina indicated. For example, the eye coverage portion 1120 can include an outer cover 1130 configured to surround the eye coverage portion 1120. The outer cover 1130 generally surrounds both the front side 1126 and back side 1128 of the eye coverage portion 1120, as shown in Figure 41. The eye coverage portion also includes an inner pouch 1136 that holds a fill material 1140, as shown in Figure 40, configured to retain moisture and deliver moist heat. The outer cover 1130 is configured to surround and contain the inner pouch 1136. The inner pouch 1136 and outer cover 1130 are preferably at least partially moisture permeable such that moisture and heat (moist heat) can travel from the fill material 1140 to the face of the patient. It may be that the eye coverage portion 1120 also includes a panel or sheet 1134 of moisture impermeable or minimally-moisture permeable material. The panel 1134 is positioned between the inner pouch 1136 and the outer cover 1130 of the eye coverage portion 1120. The panel 1134 is generally positioned towards the back or outer side 1128 of the eye coverage portion 1120 such that the panel is not positioned between the inner pouch 1136 and the face of the patient, as shown in Figure 41. The moisture impermeable panel 1134 is configured to help prevent the moisture released from the fill material 1140 from escaping through the back end 28 of the eye coverage portion 1120, and better direct moist heat to the subject for treatment. The panel 1134 can be simply held between the outer cover 1130 and the inner pouch 1136.Otherwise, the panel 1134 can be attached to the outer cover 1130, the inner pouch 1136, or another part of the eye coverage portion. Example attachment means include gluing, sewing, or other fastening means. Otherwise, it may be that the moisture barrier panel 1134 is laminated to another layer of the eye coverage portion to form a unitary structure. The panel 1134 can be flame laminated to eliminate potential for skin contact with any irritant adhesive materials. For example, the panel 1134 can be laminated to the outer rear surface of the inner pouch 1136 or the inner rear surface of the outer cover 1130.

The eye coverage portions 1120 can include an intermediate layer 1132 in between the inner pouch 1136 and the outer cover 1130, as shown in Figures 38 and 39. The intermediate layer 1132 is generally formed from a moisture permeable material. It may be that the intermediate layer 1132 surrounds and contains the inner pouch 1136. Otherwise, it may be that the intermediate layer 1132 covers only a portion of the inner pouch 1136. The moisture-barrier panel 1134 can be attached or laminated to the inner rear surface or the outer rear surface of the intermediate layer 1132.

It may be that the outer cover 1130, inner pouch 1136, and intermediate layer 1132 are constructed of a lightweight, durable, and flexible material such as foam or polyester. The material is configured to allow moist heat to reach the face of the patient, but limit the amount of moisture released from the fill material. It may also be that one or more of the layers 1130/1132/1136 are formed from an open cell thermoplastic polyurethane foam that is about 2 mm thick. In other examples, one or more of the layers 1130/1132/1136 are constructed from a 32.037 gram/liter (2 pounds per cubic foot), 0.9525 cm (3/8") thick polyether polyurethane foam. The foam layers can include polyester fabric laminated onto one side. Alternatively, one or more of the layers 1130/1132/1136 are constructed from polyester, rayon, spandex, silk, polyethylene, neoprene, ECA, ethylene-vinyl acetate (EVA), other plastic films, and/or other natural and/or synthetic fabrics or materials having the same or substantially similar characteristics and capabilities. For example, one or more of the layers 1130/1132/1136 can be formed from polyester felt. Otherwise, it may be that one or more of the layers 1130/1132/1136 are formed from a closed cell foam. The foam can be polyurethane and/or thermoformable. The outer cover 1130 can alternatively be constructed from a woven material different from the inner pouch 1136 and intermediate layer 1132. The mask material can optionally be selected to have insulative or heat-transmissive properties to affect the temperature transferred from the mask to the patient's eyes, better ensuring safety. The material may optionally be washable for reuse, or alternatively can be a single-use disposable product. It may also be that the eye coverage portions 1120 can be constructed of a material containing nanobeads comprising an antimicrobial metal, medications, and/or other therapeutic material(s). Optionally, the mask 1100 may be configured for use in connection with medicated or therapeutic sheets, pods or other therapeutic accessories positioned or retained between the eye coverage portions 1120 and the subject's eye areas. For example, the mask 1100 may be configured for supplemental delivery and transport of medication or therapeutic material with moist heat.

The moisture barrier panel or material 1134 can be constructed of a flexible material that will limit moisture that may otherwise be lost through the open cell foam, e.g., a plastic material. It may be that the panel 1134 is formed of a multilayer structure of nylon, ethylene vinyl alcohol (EVOH), and polyethylene (PE) that is about 4 mm thick, other plastic films, and/or other natural and/or synthetic fabrics or materials having the same or substantially similar characteristics and capabilities.

It may be that the fill material 1140 is loosely contained within the inner pouch 1136 and comprises a synthetic porous crystalline granular aluminosilicate zeolite - e.g., a hydrophilic natural or synthetic zeolite, also referred to as a molecular sieve material - or other substances with similar properties. In the depicted comparative example, the fill material 1140 comprises a multiplicity of individual solid beads or granules that absorb and release moisture while generally maintaining their individual solid shape and consistency. The fill material 1140 is surrounded and contained by the inner pouch 1136 such that the fill material is unable to escape from the inner pouch 1136. The fill material 1140 may further comprise other inert additives and physical matrices without affecting the antimicrobial and hydrous efficacies of the fill. The hydrophilic zeolite granules or beads are configured to repeatedly absorb and release moisture without substantially changing shape or form. Optionally, the fill material 1140 comprise a granular material such as activated alumina, silica gel, bentonite or hydrophilic zeolite or molecular sieve material loosely contained in the pouch or other enclosure. Otherwise, it may be that the fill material comprises capsules or packets of non-granular material (e.g., gel, liquid), powder, or other materials. The fill material 1140 optionally also contains a metallic or other antimicrobial agent, such as for example silver, copper, copper oxide, gold, magnesium oxide, aluminum oxide, titanium dioxide, zinc oxide, cobalt, nickel, zirconium, molybdenum, tin, lead and/or other metals; metal oxides, metal ions, metal particles or nanoparticles; and alloys, mixtures or combinations thereof deposited therein. For example, silver or other metal loading of the fill material 1140 may be attained by the process of ion-exchange. In this process, a solution containing atomic silver, or a composition of silver, bathes or is passed through a bed of the fill granules. An ion-exchange column method may be performed in which an aqueous solution containing atomic silver or a composition of silver may be passed through a column bed of the fill granules, and the eluted solution may again be passed through the bed or may receive additional silver and then be again passed through the bed.

Various ion-exchange schedules as known in the art may be applied to produce retention of the silver or other metals in the fill material 1140. For example, the final content by weight of an atomic silver or silver composition, or other metals or antimicrobial agents, may be as high as twenty percent of the final loaded fill granules. It may be that the loaded fill granules produced by ion-exchange will exhibit high retention of the silver or other metals even under subsequent exposure to fluids and microwave irradiation. The fill granules may comprise a blend of both metal loaded and unloaded (i.e., not containing metal) zeolite or other substance(s) retaining silver or other metals. The presence of the atomic silver or other metals preferably will not interfere with the useful properties of the fill granules such as the moisture desorption, absorption, and/or adsorption properties which may be desirable in the use of the eye mask or compress system. The hydrophilic nature of example forms of zeolite fill materials provides that substantial water content is available therein and readily replenished by absorption of moisture in the form of water vapor from the atmosphere or ambient surroundings at standard room temperatures and conditions. The water so absorbed may be sufficient for moist heat delivery applications, or may be supplemented by manually added water, for providing a microwave responsive water content of the eye mask or compress system. The compositions of silver or other metals used may include but are not limited to, metal compounds, and metal salts such as silver chloride and silver nitrate.

The presence of silver or other metals within the fill granules, while optional, may provide anti-microbial properties to the therapeutic eye mask system. The ion-exchange loaded fill granules will preferably retain the silver or other metals despite microwave heating as may be required in the use of the eye mask or compress system. Further, the retention of the silver or other metals within the fill granules provides assured antimicrobial performance in a reusable and potentially washable, if so desired, moist heat therapy compress. Otherwise, it may be that silver or other metals are incorporated into the cover material and/or other portions of the eye mask system, in addition to or instead of the fill granules. Alternatively, one or more non-metal antimicrobial materials and/or medications may optionally be incorporated into the fill material 1140, the eye coverage portions 1120, and/or other portions of the eye mask system.

There is also provided a method falling outside of the scope of the attached independent claim, in particular a method of providing moist heat therapy to a body part of a human or animal subject. In example forms, the method includes providing a therapeutic device comprising a first side, a second side, and a hydrophilic fill material contained between the first and second sides; wherein the first side at least partially comprises a moisture-transmissive material allowing passage of moisture therethrough for absorption and release into and from the fill material, and the second side at least partially comprises a moisture-impermeable material substantially preventing passage of moisture therethrough. It may be that the body part of the human or animal subject is an eye. It may also be that the method also includes exposing the therapeutic device to a moisture source, such as for example, water vapor in ambient air surrounding the therapeutic device to allow absorption of moisture into the hydrophilic fill material. Additionally, it may be that the method further includes exposing the therapeutic device to a heat source, such as for example, a microwave oven or other heating device to allow transfer of heat into the fill material. It may also be that the method further includes directing the application of the first side of the therapeutic device into contact with the body part to provide transmission of moist heat in a first direction from the hydrophilic fill material through the moisture-transmissive material to the body part, and to substantially prevent transmission of moist heat in an opposed second direction from the hydrophilic fill material through the moisture-impermeable material away from the body part. It may be that the method further includes applying the first side of the therapeutic device into contact with the body part to provide transmission of moist heat in a first direction from the hydrophilic fill material through the moisture-transmissive material to the body part, and to substantially prevent transmission of moist heat in an opposed second direction from the hydrophilic fill material through the moisture-impermeable material away from the body part.

The various features and embodiments disclosed herein can be used individually or in any combination(s) and/or sub-combinations thereof, and the invention includes example embodiments incorporating any one or more of the disclosed features and embodiments, and any and all permutations and combinations thereof. For example, the detachable pods configured to be secured to the compress strap can be utilized independently, or in combination with one or more of the layered compress structure, the moisture barrier panel, the moisture impermeable layer, and/or the stitched corneal relief recess in various example embodiments. The layered compress structure, the moisture barrier panel, and/or the moisture impermeable layer can be utilized independently or in combination, and/or in combination with one or more of the detachable pods and/or the stitched corneal relief recess in various example embodiments. The stitched corneal relief recess can be utilized independently, or in combination with one or more of the detachable pods and/or the layered compress structure, the moisture barrier panel, and/or the moisture impermeable layer.

While the invention has been described with reference to preferred and example embodiments, it will be understood by those skilled in the art that a variety of modifications, additions and deletions are within the scope of the invention, as defined by the following claims.

## Claims

1. An eye compress configured to deliver moist heat therapy to at least one eye of a patient, the eye compress comprising:
at least one eye coverage portion (22) having a front side and an opposite back side;
wherein the front side is configured for contact with the patient's body when the eye compress is worn; and
at least one securing strap (26) affixed to the at least one eye coverage portion:
wherein the at least one eye coverage portion at least partially comprises a moisture-permeable antimicrobial material, a fill material enclosed within the moisture-permeable antimicrobial material, and a recessed area on at least the front side of the at least one eye coverage portion, whereby the fill material is configured to absorb and release moisture through the moisture-permeable antimicrobial material and deliver moist heat therapy to the eye area of the patient.

2. The eye compress of claim 1, comprising two independent eye coverage portions and a flexible nose bridge coupled between the two eye coverage portions.

3. They eye compress of claim 2, wherein the flexible nose bridge is adjustable in length.

4. The eye compress of claim 1, wherein the fill material comprises a hydrophilic zeolite;
optionally wherein the hydrophilic zeolite fill material comprises granules loosely contained within the moisture-permeable antimicrobial material.

5. The eye compress of claim 4, wherein the fill material at least partially comprises an antimicrobial material.

6. The eye compress of claim 1, further comprising a moisture-impermeable material configured to at least partially prevent moisture transmission from the fill material in a first direction, whereby moisture from the fill material is substantially directed toward the patient in a second direction generally opposite the first direction.

7. The eye compress of claim 6, wherein the moisture-impermeable material is positioned between the fill material and the back side of the eye coverage portion.

8. The eye compress of claim 6, wherein the moisture-impermeable material is selected from a plastic material, nylon, EVOH, or PE, or other natural and/or synthetic fabrics or materials, and combinations thereof.

9. The eye compress of claim 1, wherein the moisture-permeable antimicrobial material comprises an elastic fabric, an antimicrobial fabric, and a regular fabric.

10. The eye compress of claim 9, wherein the antimicrobial fabric comprises yarn comprising silver particles.

11. The eye compress of claim 9, wherein the moisture-permeable antimicrobial material comprises 5-15% elastic fabric, 25-35% antimicrobial material, and 50-70% regular fiber.

12. The eye compress of claim 10, wherein the moisture-permeable antimicrobial material comprises 10% elastic fabric, 30% antimicrobial material, and 60% regular fiber.

13. The eye compress of claim 1, wherein the recessed area is formed by stitching together the front side and back side of the eye coverage portion.

14. The eye compress of claim 12, wherein the stitching of the recessed area extends a length of between 5 mm and 15 mm.

15. The eye compress of claim 13, wherein the length of the stitching is about 10 mm.

## Patentansprüche

1. Augenkompresse, die so konfiguriert ist, dass sie feuchte Wärmetherapie für mindestens ein Auge eines Patienten bereitstellt, wobei die Augenkompresse umfasst:
mindestens einen Augenabdeckabschnitt (22) mit einer Vorderseite und einer dahinter liegenden Rückseite,
wobei die Vorderseite für den Kontakt mit dem Körper des Patienten konfiguriert ist, wenn die Augenkompresse getragen wird, und mindestens ein Befestigungsband (26), das an dem mindestens einen Augenabdeckabschnitt befestigt ist;
wobei der mindestens eine Augenabdeckabschnitt zumindest teilweise ein feuchtigkeitsdurchlässiges antimikrobielles Material, ein in dem feuchtigkeitsdurchlässigen antimikrobiellen Material eingeschlossenes Füllmaterial und einen vertieften Bereich auf zumindest der Vorderseite des mindestens einen Augenabdeckabschnitts umfasst, wobei das Füllmaterial so konfiguriert ist, dass es Feuchtigkeit durch das feuchtigkeitsdurchlässige antimikrobielle Material absorbiert und abgibt und feuchte Wärmetherapie für den Augenbereich des Patienten bereitstellt.

2. Augenkompresse nach Anspruch 1, umfassend zwei unabhängige Augenabdeckabschnitte und einen flexiblen Nasensteg, der zwischen den beiden Augenabdeckabschnitten angebracht ist.

3. Augenkompresse nach Anspruch 2, wobei der flexible Nasensteg in der Länge verstellbar ist.

4. Augenkompresse nach Anspruch 1, wobei das Füllmaterial einen hydrophilen Zeolith umfasst, wobei das hydrophile Zeolith-Füllmaterial optional Granulat umfasst, das lose in dem feuchtigkeitsdurchlässigen antimikrobiellen Material enthalten ist.

5. Augenkompresse nach Anspruch 4, wobei das Füllmaterial zumindest teilweise ein antimikrobielles Material umfasst.

6. Augenkompresse nach Anspruch 1, ferner umfassend ein feuchtigkeitsundurchlässiges Material, das so konfiguriert ist, dass es die Feuchtigkeitsübertragung aus dem Füllmaterial in einer ersten Richtung zumindest teilweise verhindert, wodurch die Feuchtigkeit aus dem Füllmaterial im Wesentlichen in einer zweiten Richtung, die der ersten Richtung im Allgemeinen entgegengesetzt ist, auf den Patienten gerichtet wird.

7. Augenkompresse nach Anspruch 6, wobei das feuchtigkeitsundurchlässige Material zwischen dem Füllmaterial und der Rückseite des Augenabdeckabschnitts angeordnet ist.

8. Augenkompresse nach Anspruch 6, wobei das feuchtigkeitsundurchlässige Material aus einem Kunststoffmaterial, Nylon, EVOH oder PE oder anderen natürlichen und/oder synthetischen Geweben oder Materialien und Kombinationen davon ausgewählt ist.

9. Augenkompresse nach Anspruch 1, wobei das feuchtigkeitsdurchlässige antimikrobielle Material ein elastisches Gewebe, ein antimikrobielles Gewebe und ein normales Gewebe umfasst.

10. Augenkompresse nach Anspruch 9, wobei das antimikrobielle Gewebe Garn mit Silberpartikeln umfasst.

11. Augenkompresse nach Anspruch 9, wobei das feuchtigkeitsdurchlässige antimikrobielle Material 5 bis 15 % elastisches Gewebe, 25 bis 35 % antimikrobielles Material und 50 bis 70 % normale Fasern umfasst.

12. Augenkompresse nach Anspruch 10, wobei das feuchtigkeitsdurchlässige antimikrobielle Material 10 % elastisches Gewebe, 30 % antimikrobielles Material und 60 % normale Fasern umfasst.

13. Augenkompresse nach Anspruch 1, wobei der vertiefte Bereich durch Zusammennähen der Vorderseite und der Rückseite des Augenabdeckabschnitts gebildet wird.

14. Augenkompresse nach Anspruch 12, wobei die Naht des vertieften Bereichs eine Länge zwischen 5 mm und 15 mm aufweist.

15. Augenkompresse nach Anspruch 13, wobei die Länge der Naht etwa 10 mm beträgt.

## Revendications

1. Compresse oculaire configurée pour délivrer une thérapie par chaleur humide à au moins un œil d'un patient, la compresse oculaire comprenant :
au moins une partie de recouvrement d'œil (22) ayant un côté avant et un côté arrière opposé, le côté avant étant configuré pour être en contact avec le corps du patient lorsque la compresse oculaire est portée ; et
au moins une sangle de fixation (26) fixée à l'au moins une partie de recouvrement d'œil ;
l'au moins une partie de recouvrement d'œil comprenant au moins partiellement un matériau antimicrobien perméable à l'humidité, un matériau de remplissage enfermé dans le matériau antimicrobien perméable à l'humidité et une zone évidée sur au moins le côté avant de l'au moins une partie de recouvrement d'œil, moyennant quoi le matériau de remplissage est configuré pour absorber et libérer l'humidité à travers le matériau antimicrobien perméable à l'humidité et fournir une thérapie de chaleur humide à la zone oculaire du patient.

2. Compresse oculaire selon la revendication 1, comprenant deux parties de recouvrement d'œil indépendantes et un pont nasal flexible couplé entre les deux parties de recouvrement d'œil.

3. Compresse oculaire selon la revendication 2, le pont nasal flexible étant réglable en longueur.

4. Compresse oculaire selon la revendication 1, le matériau de remplissage comprenant une zéolite hydrophile ;
facultativement, le matériau de remplissage de zéolite hydrophile comprenant des granulés contenus de manière lâche dans le matériau antimicrobien perméable à l'humidité.

5. Compresse oculaire selon la revendication 4, le matériau de remplissage comprenant au moins partiellement un matériau antimicrobien.

6. Compresse oculaire selon la revendication 1, comprenant en outre un matériau imperméable à l'humidité configuré pour empêcher au moins partiellement la transmission de l'humidité depuis le matériau de remplissage dans une première direction, moyennant quoi l'humidité provenant du matériau de remplissage est sensiblement dirigée vers le patient dans une deuxième direction généralement opposée à la première direction.

7. Compresse oculaire selon la revendication 6, le matériau imperméable à l'humidité étant positionné entre le matériau de remplissage et le côté arrière de la partie de recouvrement d'œil.

8. Compresse oculaire selon la revendication 6, le matériau imperméable à l'humidité étant choisi parmi un matériau plastique, le nylon, l'EVOH ou le PE, ou d'autres tissus ou matériaux naturels et/ou synthétiques, et leurs combinaisons.

9. Compresse oculaire selon la revendication 1, le matériau antimicrobien perméable à l'humidité comprenant un tissu élastique, un tissu antimicrobien et un tissu ordinaire.

10. Compresse oculaire selon la revendication 9, le tissu antimicrobien comprenant un fil comprenant des particules d'argent.

11. Compresse oculaire selon la revendication 9, le matériau antimicrobien perméable à l'humidité comprenant 5 à 15 % de tissu élastique, 25 à 35 % de matériau antimicrobien, et 50 à 70 % de fibres ordinaires.

12. Compresse oculaire selon la revendication 10, le matériau antimicrobien perméable à l'humidité comprenant 10 % de tissu élastique, 30 % de matériau antimicrobien et 60 % de fibres ordinaires.

13. Compresse oculaire selon la revendication 1, la zone évidée étant formée en cousant ensemble le côté avant et le côté arrière de la partie de recouvrement d'œil.

14. Compresse oculaire selon la revendication 12, la couture de la zone évidée s'étendant sur une longueur comprise entre 5 mm et 15 mm.

15. Compresse oculaire selon la revendication 13, la longueur de la couture étant d'environ 10 mm.
